(19) Europäisches Patentamt
European Patent Office
Office européen des brevets

(11) **EP 1 203 087 B1**

(12) **EUROPÄISCHE PATENTSCHRIFT**

(45) Veröffentlichungstag und Bekanntmachung des Hinweises auf die Patenterteilung:
**07.01.2009 Patentblatt 2009/02**

(21) Anmeldenummer: **00954587.2**

(22) Anmeldetag: **04.08.2000**

(51) Int Cl.:
*C12N 15/82* (2006.01)       *C12N 9/10* (2006.01)
*C12N 15/56* (2006.01)       *C12N 5/14* (2006.01)
*A23L 1/0522* (2006.01)      *A01H 5/00* (2006.01)

(86) Internationale Anmeldenummer:
**PCT/EP2000/007575**

(87) Internationale Veröffentlichungsnummer:
**WO 2001/012782 (22.02.2001 Gazette 2001/08)**

(54) **TRANSGENE PFLANZENZELLEN UND PFLANZEN MIT VERÄNDERTER AKTIVITÄT DES GBSSI- UND DES BE-PROTEINS**

TRANSGENICALLY MODIFIED PLANT CELLS AND PLANTS HAVING MODIFIED GBSSI- AND BE-PROTEIN ACTIVITY

CELLULES VEGETALES ET PLANTES TRANSGENIQUES A ACTIVITE MODIFIEE DES PROTEINES GBSSI ET BE

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **12.08.1999 DE 19937643**

(43) Veröffentlichungstag der Anmeldung:
**08.05.2002 Patentblatt 2002/19**

(73) Patentinhaber: **Bayer CropScience AG
40789 Monheim (DE)**

(72) Erfinder: **LANDSCHÜTZE, Volker
D-12203 Berlin (DE)**

(74) Vertreter: **Beyer, Andreas et al
Bayer BioScience GmbH
Intellectual Property Department
Hermannswerder 20a
14473 Potsdam (DE)**

(56) Entgegenhaltungen:
WO-A-97/45545          DE-A- 19 618 125
DE-A- 19 653 176       DE-A- 19 836 098
US-A- 5 856 467

- VISSER R G F ET AL: "INHIBITION OF THE EXPRESSION OF THE GENE FOR GRANULE-BOUND STARCH SYNTHASE IN POTATO BY ANTISENSE CONSTRUCTS" MOLECULAR AND GENERAL GENETICS,DE,SPRINGER VERLAG, BERLIN, Bd. 225, 1. Februar 1991 (1991-02-01), Seiten 289-296, XP000611638 ISSN: 0026-8925
- CHAKRABORTY M ET AL.: "Physicochemical and functional properties of tetraploid and hexaploid waxy wheat starch" STARCH, Bd. 56, 2004, Seiten 339-347,
- YOO SANG-HO & JANE JAY-LIN: "Structural and physical characteristics of waxy and other wheat starches" CARBOHYDRATE POLYMERS, Bd. 49, 2002, Seiten 297-305, XP004350808
- YOSHIMOTO Y ET AL.: "Molecular structure and some physicochemical properties of waxy and low-amylose barley starches" CARBOHYDRATE POLYMERS, Bd. 47, 2002, Seiten 159-167, XP004323707

EP 1 203 087 B1

**Beschreibung**

Die vorliegende Erfindung betrifft transgene Kartoffel-Pflanzenzellen und -Pflanzen mit einer verringerten Aktivität eines GBSSI-Proteins und einer verringerten Aktivität eines BE-Proteins, insbesondere eines BEI-Proteins, sowie Mittel und Verfahren zu deren Herstellung. Derartige Kartoffel-Pflanzenzellen und -Pflanzen können eine modifizierte Stärke synthetisieren, die dadurch gekennzeichnet ist, daß sie einen Amylopektingehalt von mindestens 90% und einen im Vergleich zu Stärke aus entsprechenden Kartoffel-Pflanzen des waxy-Phänotyps erhöhten Phosphatgehalt und/oder eine verringerte Verkleisterungstemperatur aufweist. Die vorliegende Erfindung betrifft daher auch Verfahren zur Herstellung einer Stärke, die von den erfindungsgemäßen Kartoffel-Pflanzenzellen und -Pflanzen synthetisierte wird. Ferner betrifft die vorliegende Erfindung die Verwendung bestimmter Nucleinsäuremoleküle zur Herstellung von Kartoffel-Pflanzen, die eine Kartoffel-Stärke mit einem Amylopektingehalt von mindestens 90% synthetisieren, die im Vergleich zu Stärke aus entsprechenden Kartoffel-Pflanzen des waxy-Phänotyps eine verringerte Verkleisterungstemperatur aufweist.

Im Hinblick auf die zunehmende Bedeutung, die pflanzlichen Inhaltsstoffen als erneuerbaren Rohstoffquellen in letzter Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in höheren Pflanzen ist. Hierbei ist Mais eine der interessantesten Pflanzen, da sie die weltweit für die Stärkeproduktion wichtigste Kulturpflanze ist.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades und des Auftretens von Verzweigungen der Glucoseketten unterscheiden. Daher stellt Stärke keinen einheitlichen Rohstoff dar. Man unterscheidet insbesondere die Amylose-Stärke, ein im wesentlichen unverzweigtes Polymer aus $\alpha$-1,4-glycosidisch verknüpften Glucosemolekülen, von der Amylopektin-Stärke, die ihrerseits ein komplexes Gemisch aus unterschiedlich verzweigten Glucoseketten darstellt. Die Verzweigungen kommen dabei durch das Auftreten von zusätzlichen $\alpha$-1,6-glycosidischen Verknüpfungen zustande. In typischen für die Stärkeproduktion verwendeten Pflanzen, wie z.B. Mais oder Kartoffel, besteht die synthetisierte Stärke zu ca. 20% - 30% aus Amylose-Stärke und zu ca. 70% - 80% aus Amylopektin-Stärke.

Um eine möglichst breite Anwendung von Stärke zu ermöglichen, erscheint es wünschenswert, Pflanzen zur Verfügung zu stellen, die in der Lage sind, modifizierte Stärke zu synthetisieren, die sich für verschiedene Verwendungszwecke besonders eignet. Eine Möglichkeit, derartige Pflanzen bereitzustellen, besteht - neben züchterischen Maßnahmen - in der gezielten genetischen Veränderung des Stärkemetabolismus stärkeproduzierender Pflanzen durch gentechnologische Methoden.

Das Verhältnis von Amylopektin zu Amylose hat einen starken Einfluß auf die physiko-chemischen Eigenschaften der Stärken und somit auf die jeweiligen Anwendungsmöglichkeiten dieser Stärken. Da Verfahren zur Trennung dieser beiden Komponenten sehr zeitaufwendig und kostenintensiv sind, werden derartige Verfahren großtechnisch nicht mehr angewendet (Young, A.H. in: Starch Chemistry and Technology. Eds. R. L. Whistler, J. N. BeMiller and E. F. Paschall. Academic Press, New York, 1984, 249-283). Für eine Vielzahl von Anwendungen wäre es somit wünschenswert, Stärken zur Verfügung zu haben, die nur noch eines der beiden Polymere enthalten.

Bisher sind sowohl Mutanten als auch durch gentechnologische Verfahren erzeugte Pflanzen beschrieben worden, die ein im Vergleich zu entsprechenden Wildtyppflanzen verändertes Amylopektin/Amylose-Verhältnis aufweisen. Beispielsweise produziert eine sogenannte "waxy"-Mutante aus Mais, die eine Mutation im Gen codierend für die stärkekorngebundene-Stärkesynthase I (granule bound starch synthase I, abgekürzt: GBSSI) (Akasuka und Nelson, J. Biol. Chem., 241, (1966), 2280-2285; Shure et al., Cell 35 (1983), 225-233) aufweist, eine Stärke, die im wesentlichen aus Amylopektin besteht. Im Folgenden soll unter einer waxy-Stärke eine Stärke mit einem Amylopektingehalt von mindestens 90% verstanden werden.

Für Kartoffel wurden sowohl durch chemische Mutagenese einer haploiden Linie (Hovenkamp-Hermelink et al., Theor. Appl. Genet., 225, (1987), 217-221) als auch durch antisense-Inhibierung des Gens für die GBSSI Genotypen erzeugt, deren Stärken im wesentlichen aus Amylopektinstärke bestehen. Derartige waxy-Kartoffelstärken weisen im Vergleich zu Stärken aus entsprechenden Wildtyppflanzen keine Unterschiede hinsichtlich des Phosphatgehalts, in der Morphologie des Stärkekorns oder im Ionengehalt auf (Visser et al., Starch/Stärke, 49, (1997), 438-443).

Die funktionellen Eigenschaften der Stärke werden neben dem Amylose/Amylopektin-Verhältnis stark beeinflußt durch den Phosphatgehalt, das Molekulargewicht, das Muster der Seitenkettenverteilung, den Gehalt an Ionen, den Lipid- und Proteingehalt etc.. Als wichtige funktionelle Eigenschaften sind hierbei beispielsweise zu nennen die Löslichkeit, das Retrogradierungsverhalten, das Wasserbindevermögen, die Filmbildungseigenschaften, die Viskosität, die Verkleisterungseigenschaften, die Stabilität etc.. Auch die Stärkekomgröße kann für verschiedene Anwendungen von Bedeutung sein.

Der Phosphatgehalt läßt sich grundsätzlich sowohl durch gentechnische Ansätze (s. beispielsweise WO 97/11188-A1; Safford et al., Carbohydrate Polymers 35, (1998), 155-168) als auch durch nachträgliche chemische Phosphorylierung (s. beispielsweise in: Starch Chemistry and Technology. Eds. R. L. Whistler, J. N. BeMiller and E. F. Paschall. Academic Press, New York, 1988, 349-364) modifizieren. Chemische Modifikationen sind jedoch in der Regel kosten- und zeitintensiv.

Pflanzenzellen und Pflanzen, die waxy-Stärken mit im Vergleich zu Stärken aus entsprechenden Pflanzen (zellen) des waxy-Phänotyps erhöhtem Phosphatgehalt und/oder verringerter Verkleisterungstemperatur synthetisieren, konnten bisher nicht erzeugt werden. Auch Verfahren zur Herstellung derartiger Pflanzenzellen und Pflanzen sowie Verfahren zur Herstellung derartiger Stärken sind im Stand der Technik bisher nicht beschrieben.

Da der Phosphatgehalt der Stärken deren Eigenschaften beeinflußt, wäre es wünschenswert, Pflanzenzellen und Pflanzen zur Verfügung zu stellen, die waxy-Stärken mit im Vergleich zu entsprechenden Pflanzenzellen und Pflanzen des waxy-Phänotyps veränderten strukturellen und/oder funktionellen Eigenschaften synthetisieren.

Somit liegt der vorliegenden Erfindung die Aufgabe zugrunde, Kartoffel-Pflanzenzellen und -Pflanzen zur Verfügung zu stellen, welche im Vergleich zu entsprechenden Kartoffel-Pflanzenzellen und-Pflanzen des waxy-Phänotyps Stärken mit veränderten strukturellen und/oder funktionellen Eigenschaften synthetisieren, sowie Kartoffel-waxy-Stärke zur Verfügung zu stellen, welche sich in ihren strukturellen und/oder funktionellen Eigenschaften von anderen waxy-Stärken unterscheidet und somit für allgemeine und/oder spezielle Verwendungszwecke besser geeignet ist.

Diese Aufgabe wird durch die Bereitstellung der in den Patentansprüchen bezeichneten Ausführungsformen gelöst.

Somit beschreibt die vorliegende Erfindung transgene Kartoffel-Pflanzenzellen, die genetisch modifiziert sind, wobei die genetische Modifikation zur Verringerung der Aktivität eines oder mehrerer endogen in der Kartoffel-Pflanzenzelle vorkommenden GBSSI-Proteine und zur Verringerung der Aktivität eines oder mehrerer endogen in der Kartoffel-Pflänzenzelle vorkommenden BE-Proteine führt, im Vergleich zu entsprechenden nicht genetisch modifizierten Pflanzenzellen von Kartoffel Wildtyppflanzen.

Die genetische Modifikation kann dabei jede genetische Modifikation sein, die zu einer Verringerung der Aktivität eines endogen in der Kartoffel-Pflanzenzelle vorkommenden GBSSI-Proteins und eines BE-Proteins führt im Vergleich zu nicht genetisch modifizierten Pflanzenzellen entsprechender Kartoffel-Wildtyppflanzen.

Der Begriff "transgen" bedeutet in diesem Zusammenhang, daß die erfindungsgemäßen Kartoffel-Pflanzenzellen aufgrund einer genetischen Modifikation, insbesondere der Einführung eines oder mehrerer fremder Nucleinsäuremoleküle in ihrer genetischen Information von entsprechenden nicht genetisch modifizierten Kartoffel-Pflanzenzellen abweichen.

Der Begriff "genetisch modifiziert" bedeutet dabei in diesem Zusammenhang, daß die Kartoffel-Pflanenzelle durch Einführung eines oder mehrerer fremder Nucleinsäuremoleküle in ihrer genetischen Information verändert ist und daß das Vorhandensein oder die Expression des fremden Nucleinsäuremoleküls zu einer phänotypischen Veränderung führt. "Phänotypische Veränderung" bedeutet dabei vorzugsweise eine meßbare Veränderung einer oder mehrerer Funktionen der Zellen, insbesondere zeigen erfindungsgemäße Kartoffel-Pflanzenzellen eine Verringerung der Expression mindestens eines endogenen GBSSI- und mindestens eines endogenen BE-Gens und/oder eine Verringerung der Aktivität mindestens eines GBSSI-Proteins sowie mindestens eines BE-Proteins.

Unter dem Begriff "GBSSI-Protein" versteht man im Rahmen der vorliegenden Erfindung jedes Protein, das im Unterschied zur Klasse der löslichen Stärkesynthasen zur Klasse der stärkekorngebundenen Stärkesynthasen ("granulebound starch synthases") Isoform I (=GBSSI, EC 2.4.1.21) gehört. Pflanzen, in denen die Enzymaktivität dieses Proteins stark oder vollkommen reduziert ist, synthetisieren eine im wesentlichen amylosefreie, sogenannte waxy-Stärke (Shure et al., (1983) s.o.; Hovenkamp-Hermelink et al., (1987), s.o.; Visser et al., Mol. Gen. Genet., 225, (1991), 289-296), so daß diesem Enzym eine entscheidende Rolle bei der Synthese der Amylosestärke zugeschrieben wird. Nucleinsäuremoleküle, die für GBSSI-Proteine codieren, sind für zahlreiche Pflanzen beschrieben worden, beispielsweise Mais (Genbank Acc. No. AF079260, AF079261), Weizen (Genbank Acc. No. AB019622, AB019623, AB019624), Reis (Genbank Acc. No. AF092443, AF092444, AF031162), Kartoffel (Genbank Acc. No. X58453), Gerste (Genbank Acc. No. X07931, X07932). Mit Hilfe dieser bekannten Nucleinsäuremoleküle ist es dem Fachmann möglich nach Standardverfahren, beispielsweise durch heterologes Screening, entsprechende Sequenzen aus anderen Organismen, insbesonderen pflanzlichen zu isolieren.

Im Rahmen der vorliegenden Erfindung wird unter einem Verzweigungsenzym oder BE-Protein ($\alpha$-1,4-Glucan: $\alpha$-1,4-Glucan 6-Glycosyltransferase, E.C. 2.4.1.18) ein Protein verstanden, das eine Transglycosylierungsreaktion katalysiert, in der $\alpha$-1,4-Verknüpfungen eines $\alpha$-1,4-Glucandonors hydrolysiert und die dabei freigesetzten $\alpha$-1,4-Glucanketten auf eine $\alpha$-1,4-Glucanakzeptorkette transferiert und dabei in $\alpha$-1,6-Verknüpfungen überführt werden, vorzugsweise ein BEI-Protein.

Der Begriff "BEI-Protein" bezeichnet ein Verzweigungsenzym (branching enzyme = BE) der Isoform I. Die Bezeichnung der Isoformen lehnt an der von Smith-White und Preiss vorgeschlagenen Nomenklatur an (Smith-White & Preiss, Plant Mol Biol. Rep. 12, (1994), 67-71, Larsson et al., Plant Mol Biol. 37, (1998), 505-511). Im Zusammenhang mit der vorliegenden Erfindung sollen alle Enzyme, die dem BEI-Protein aus Mais (Baba et al., Biochem. Biophys. Res. Commun. 181 (1), (1991), 87-94; Kim et al. Gene 216, (1998), 233-243) strukturell, d. h. auf Ebene der Aminosäuresequenz, ähnlicher sind als der BEII-Isoform des Proteins aus Mais (Genbank Acc. No AF072725 , U65948) als Isoform I bezeichnet werden. In Kartoffelpflanzen wird das BEI-Gen hauptsächlich in den Knollen und kaum in den Blättern exprimiert (Larsson et al., Plant. Mol. Biol. 37, (1998), 505-511).

Nucleinsäuremoleküle, die für ein BEI-Protein codieren, sind für zahlreiche Pflanzen beschrieben worden, beispielsweise für Mais (Genbank Acc. No. D 11081, AF 072724), Reis Genbank Acc. No. D11082), Kartoffel (verschiedene Formen des BEI-Gens(-Proteins) aus Kartoffel wurden beispielsweise beschrieben bei Khoshnoodi et al., Eur. J. Biochem. 242 (1),148-155 (1996); Genbank Acc. No. Y 08786, Kossmann et al., Mol. Gen. Genet. 230, (1991), 39-44), Erbse (Genbank Acc. No. X80010). Mit Hilfe dieser bekannten Nucleinsäuremoleküle ist es dem Fachmann möglich nach Standardverfahren, beispielsweise durch heterologes Screening, entsprechende Sequenzen aus anderen Organismen, insbesonderen pflanzlichen zu isolieren.

Ferner beschreibt die vorliegende Erfindung transgene Kartoffel-Pflanzenzellen, die genetisch modifiziert sind, wobei die genetische Modifikation in der Einführung eines oder mehrerer fremder Nucleinsäuremoleküle besteht, deren Vorhandensein und/oder Expression zur Verringerung der Aktivität von GBSSI- und BE-Proteinen führt, im Vergleich zu entsprechenden nicht genetisch modifizierten Pflanzenzellen von Kartoffel-Wildtyppflanzen.

Die Herstellung derartiger erfindungsgemäßer Kartoffel-Pflanzenzellen mit verringerter Aktivität eines GBSSI- und eines BE-Proteins kann durch verschiedene, dem Fachmann bekannte Verfahren erzielt werden, z.B. durch solche, die zu einer Inhibierung der Expression endogener Gene führen, die ein GBSSI-Protein bzw. ein BE-Protein codieren. Hierzu zählen beispielsweise die Expression einer entsprechenden antisense-RNA, die Bereitstellung von Molekülen oder Vektoren, die einen Cosuppressionseffekt vermitteln, die Expression eines entsprechend konstruierten Ribozyms, das spezifisch Transkripte spaltet, die ein GBSSI-Protein bzw. ein BE-Protein codieren, oder die sogenannte "in-vivo-Mutagenese". Alle diese Verfahren basieren auf der Einführung eines oder mehrerer fremder Nucleinsäuremoleküle in das Genom von Pflanzenzellen.

Unter dem Begriff "fremdes Nucleinsäuremolekül" versteht man ein solches Nucleinsäuremolekül, das entweder natürlicherweise in entsprechenden Kartoffel-Pflanzenzellen nicht vorkommt, oder das in der konkreten räumlichen Anordnung nicht natürlicherweise in den Kartoffel-Pflanzenzellen vorkommt oder das an einem Ort im Genom der Kartoffel-Pflanzenzelle lokalisiert ist, an dem es natürlicherweise nicht vorkommt. Bevorzugt ist das fremde Nucleinsäuremolekül ein rekombinantes Nucleinsäuremolekül, das aus verschiedenen Elementen besteht, deren Kombination oder spezifische räumliche Anordnung natürlicherweise in pflanzlichen Kartoffel-Zellen nicht auftritt.

Das "fremde Nucleinsäuremolekül" kann beispielsweise ein sogenanntes "Doppelkonstrukt" sein, worunter man einen einzigen Vektor zur Pflanzentransformation versteht, der sowohl die genetische Information zur Inhibierung der Expression eines oder mehrere endogenen GBSSI-Gene als auch zur Inhibierung der Expression eines oder mehrere BE-Gene enthält oder dessen Vorhandensein und/oder Expression zur Verringerung der Aktivität eines oder mehrerer GBSSI- und eines oder mehrere BE-Proteine führt.

In einer weiteren Ausführungsform der Erfindung wird in das Genom der Kartoffel-Pflanzenzelle nicht nur ein einziger spezifischer Vektor, sondern mehrere unterschiedliche fremde Nucleinsäuremoleküle eingeführt, wobei eines dieser fremden Nucleinsäuremoleküle ein DNA-Molekül ist, das z.B. ein Cosuppressions-Konstrukt darstellt, das eine Verringerung der Expression von endogenen GBSSI-Genen bewirkt, und ein weiteres fremdes Nucleinsäuremolekül ein DNA-Molekül ist, das z.B. eine antisense-RNA codiert, die eine Verringerung der Expression von endogenen BE-Genen bewirkt. Grundsätzlich ist bei der Konstruktion der fremden Nucleinsäuremoleküle aber auch die Verwendung jeder Kombination aus Antisense-, Cosuppressions- und Ribozymkonstrukten oder in-vivo-mutagenese geeignet, die zu einer gleichzeitigen Verringerung der Genexpression endogener GBSSI- und BE-Gene oder die zu einer gleichzeitigen Verringerung der Aktiviät von GBSSI- und BE-Proteinen, in der genetisch modifizierten Kartoffel-Pflanzenzelle führen.

Die fremden Nucleinsäuremoleküle können hierbei zeitgleich ("Cotransformation") oder nacheinander, d.h. zeitlich aufeinanderfolgend ("Supertransformation") in das Genom der Kartoffel-Pflänzenzelle eingeführt werden. Mehrere fremde Nukleinsäuremoleküle können z.B. in einem "Doppelkonstrukt" in kombinierter Form enthalten sein.

In einer Ausführungsform der Erfindung wird zur Reduzierung der Aktivität eines oder mehrerer GBSSI-Proteine und/oder eines oder mehrerer BE-Proteine, in pflanzlichen Kartoffel-Zellen mindestens eine antisense-RNA exprimiert.

Hierzu kann beispielsweise ein DNA-Molekül verwendet werden, das die gesamte für ein GBSSI-Protein und/ oder ein BE-Protein codierende Sequenz einschließlich eventuell vorhandener flankierender Sequenzen umfaßt, als auch DNA-Moleküle, die nur Teile der codierenden Sequenz umfassen, wobei diese Teile lang genug sind, um in den Zellen einen antisense-Effekt zu bewirken. Geeignet sind im allgemeinen Sequenzen bis zu einer Mindestlänge von 15 bp, vorzugsweise einer Länge von 100-500 bp, für eine effiziente antisense-Inhibition, insbesondere Sequenzen mit einer Länge über 500 bp. In der Regel werden hierfür DNA-Moleküle verwendet, die kürzer als 5000 bp, vorzugsweise Sequenzen, die kürzer als 2500 bp sind.

Möglich ist auch die Verwendung von DNA-Sequenzen, die einen hohen Grad an Homologie zu den endogen in der Kartoffel-Pflanzenzelle vorkommenden Sequenzen haben, die ein GBSSI-Protein bzw. ein BE-Protein codieren. Die minimale Homologie sollte größer als ca. 65 % sein. Die Verwendung von Sequenzen mit Homologien zwischen 95 und 100 % ist bevorzugt.

In einer weiteren Ausführungsform wird die Verringerung der GBSSI- und/oder der BE-Aktivität in den Kartoffel-Pflanzenzellen durch einen Cosuppressionseffekt erzielt. Das Verfahren ist dem Fachmann bekannt und ist beispielsweise beschrieben in Jorgensen (Trends Biotechnol. 8 (1990), 340-344), Niebel et al., (Curr. Top. Microbiol. Immunol. 197 (1995), 91-103), Flavell et al. (Curr. Top. Microbiol. Immunol. 197 (1995), 43-46), Palaqui und Vaucheret (Plant. Mol. Biol. 29 (1995), 149-159), Vaucheret et al., (Mol. Gen. Genet. 248 (1995), 311-317), de Borne et al. (Mol. Gen. Genet. 243 (1994), 613-621). Wie im Falle der antisense-Technologie können sowohl DNA-Moleküle verwendet werden, die für die gesamte codierende Region des GBSSI und/oder des BE-Proteins, codieren als auch DNA-Moleküle, die nur Teile der codierenden Sequenz umfassen.

Geeignet ist auch die Verwendung von DNA-Sequenzen, die einen hohen Grad an Homologie zu den endogen in der Kartoffel-Pflanzenzelle vorkommenden Sequenzen haben, die GBSSI- und/oder BE-Proteine codieren. Die minimale Homologie sollte größer als ca. 65 % sein. Die Verwendung von Sequenzen mit Homologien zwischen 95 und 100 % ist bevorzugt. Beispielsweise wird zur Inhibierung des BEI-Gens aus Kartoffel vorzugsweise eine DNA-Sequenz codierend für ein BEI-Protein verwendet, insbesondere aus Kartoffel, Kossmann et al. (Mol. Gen. Genet. 230, (1991), 39-44).

Die Expression von Ribozymen zur Verringerung der Aktivität von bestimmten Enzymen in Zellen ist dem Fachmann bekannt und ist beispielsweise beschrieben in EP-B1 0321201. Die Expression von Ribozymen in pflanzlichen Zellen wurde z.B. beschrieben in Feyter et al. (Mol. Gen. Genet. 250, (1996), 329-338).

Ferner kann die Verringerung der GBSSI- und/oder der BE-Aktivität, in den erfindungsgemäßen Kartoffel-Pflanzenzellen auch durch die sogenannte "in vivo-Mutagenese" erreicht werden, bei der durch Transformation von Zellen ein hybrides RNA-DNA-Oligonucleotid ("Chimeroplast") in Zellen eingeführt wird (Kipp, P.B. et al., Poster Session beim " 5th International Congress of Plant Molecular Biology, 21.-27. September 1997, Singapore; R. A. Dixon und C.J. Amtzen, Meeting report zu "Metabolic Engineering in Transgenic Plants", Keystone Symposia, Copper Mountain, CO, USA, TIBTECH 15, (1997), 441-447; internationale Patentanmeldung WO 9515972-A1; Kren et al., Hepatology 25, (1997), 1462-1468; Cole-Strauss et al., Science 273, (1996), 1386-1389).

Ein Teil der DNA-Komponente des RNA-DNA-Oligonucleotids ist homolog zu einer Nucleinsäuresequenz eines endogenen GBSSI-Gens und/oder BE-Gens, weist jedoch im Vergleich zur Nucleinsäuresequenz des endogenen GBSSI-Gens und/oder BE-Gens eine Mutation auf oder enthält eine heterologe Region, die von den homologen Regionen umschlossen ist.

Durch Basenpaarung der homologen Regionen des RNA-DNA-Oligonucleotids und des endogenen Nucleinsäuremoleküls, gefolgt von homologer Rekombination kann die in der DNA-Komponente des RNA-DNA-Oligonucleotids enthaltene Mutation oder heterologe Region in das Genom einer Pflanzenzelle übertragen werden. Dies führt zu einer Verringerung der Aktivität eines GBSSI-Proteins und/oder eines BE-Proteins. Ferner ist dem Fachmann bekannt, daß er die Aktivität eines GBSSI-Proteins und/oder eines BE-Proteins durch die Expression von nicht-funktionellen Derivaten insbesondere trans-dominanten Mutanten solcher Proteine und/oder durch die Expression von Antagonisten/Inhibitoren solcher Proteine erreichen kann. Antagonisten/Inhibitoren solcher Proteine umfassen beispielsweise Antikörper, Antikörperfragmente oder Moleküle mit ähnlichen Bindungseigenschaften. Beispielsweise wurde ein cytoplasmatischer scFv Antikörper eingesetzt um die Aktivität des Phytochrom A Proteins in gentechnisch veränderten Tabakpflanzen zu modulieren (Owen, Bio/Technology 10 (1992), 790-4; Review: Franken, E, Teuschel, U. und Hain, R., Current Opinion in Biotechnology 8, (1997), 411-416; Whitelam, Trends Plant Sci. 1 (1996), 268-272).

Die vorliegende Erfindung beschreibt transgene Kartoffel-Pflanzenzellen mit einer im Vergleich zu unmodifizierten Kartoffel-Pflanzenzellen verringerten Aktivität endogener GBSSI und BE-Proteine, die eines oder mehrere fremde Nucleinsäuremoleküle enthalten, ausgewählt aus der Gruppe bestehend aus

a) DNA-Molekül, das zur Synthese mindestens einer antisense-RNA führt, welche eine Verringerung der Expression von endogenen Genen, die ein GBSSI- und/oder ein BE-Protein codieren, bewirkt;

b) DNA-Molekül, das über einen Cosuppressionseffekt zu einer Verringerung der Expression von endogenen Genen, die ein GBSSI- und/oder ein BE-Proteincodieren, führt;

c) DNA-Molekül, das zur Synthese mindestens eines Ribozyms führt, welches spezifisch Transkripte von Genen, die ein GBSSI- und/oder ein BE-Protein codieren, spaltet; und

d) Nukleinsäuremolekül, das aufgrund in-vivo-Mutagenese zu einer Mutation oder Insertion einer heterologen Nukleinsäure-Sequenz in mindestens einem endogenen GBSSI- und/oder BE-Protein codierenden Gen führt, wobei die Mutation oder Insertion eine Verringerung der Expression des GBSSI-Gens und/oder des BE-Gens, oder die Synthese eines inaktiven GBSSI- und/oder inaktiven BE-Proteins bewirkt.

Der Begriff "Verringerung der Aktivität" bedeutet dabei im Rahmen der vorliegenden Erfindung eine Verringerung der Expression endogener Gene, die ein GBSSI- und ein BE-Protein codieren, eine Verringerung der Menge an GBSSI- und BE-Protein in den Kartoffel-Zellen und/ oder eine Verringerung der enzymatischen Aktivität des GBSSI- und des BE-Proteins in den Kartoffel-Zellen.

Die Verringerung der Expression kann beispielsweise bestimmt werden durch Messung der Menge an GBSSI- und BE-Protein codierenden Transkripten, z.B. durch Northern-Blot-Analyse. Eine Verringerung bedeutet dabei vorzugsweise eine Verringerung der Menge an Transkripten im Vergleich zu entsprechenden nicht genetisch modifizierten Kartoffel-Zellen um mindestens 50%, bevorzugt um mindestens 70%, besonders bevorzugt um mindestens 85% und ganz besonders bevorzugt um mindestens 95%.

Die Verringerung der Menge an GBSSI- und BE-Protein kann beispielsweise bestimmt werden durch Western-Blot-Analyse. Eine Verringerung bedeutet dabei vorzugsweise eine Verringerung der Menge an GBSSI- und BE-Protein im Vergleich zu entsprechenden nicht genetisch modifizierten Kartoffel-Zellen um mindestens 50%, bevorzugt um mindestens 70%, besonders bevorzugt um mindestens 85% und ganz besonders bevorzugt um mindestens 95%.

Die Verringerung der enzymatischen Aktivität des GBSSI-Proteins kann beispielweise bestimmt werden durch die von Kuipers et al., Plant Mol. Biol., 26 (1994), 1759-1773 beschriebene Methode. Die Verringerung der enzymatischen Aktivität des BE-Proteins kann bestimmt werden durch die von Safford et al., Carbohydrate Polymers 35, (1998), 155-168 beschriebene Methode. Eine Verringerung der enzymatischen Aktiviät im Vergleich zu entsprechenden nicht genetisch modifizierten Kartoffel-Zellen bedeutet dabei vorzugsweise eine Verringerung um mindestens 50%, bevorzugt um mindestens 70%, besonders bevorzugt um mindestens 85% und ganz besonders bevorzugt um mindestens 95%.

Die erfindungsgemäßen transgenen Kartoffel-Pflanzenzellen synthetisieren eine modifizierte Stärke, die beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Viskositätsverhalten, der Stärkekorngröße und/oder der Stärkekornform im Vergleich zu in Kartoffel-Wildtyppflanzen synthetisierter Stärke verändert sein kann, so daß diese für spezielle Verwendungszwecke besser geeignet ist.

Es wurde überraschenderweise gefunden, daß bei kartoffel-Pflanzenzellen, bei denen die Aktivität des GBSSI- und des BEI-Proteins verringert ist, die Zusammensetzung der Stärke in der Weise verändert ist, daß sie nicht nur durch einen Amylopektingehalt von mindestens 90%, sondern zusätzlich auch durch einen erhöhten Phosphatgehalt im Vergleich zu Kartoffel-Pflanzenzellen von entsprechenden Kartoffel-Pflanzen des waxy-Phänotyps gekennzeichnet ist. Dieser erhöhte Phosphatgehalt wirkt sich auf die funktionellen Eigenschaften der Stärke aus, so daß diese für spezielle Verwendungszwecke besser geeignet ist.

Die vorliegenden Erfindung beschreibt auch transgene Kartoffel-Pflanzenzellen, die eine modifizierte Stärke mit einem Amylopektingehalt von mindestens 90%, bevorzugt von mindestens 93%, besonders bevorzugt von mindestens 95% und insbesondere bevorzugt von mindestens 97% enthalten und im Vergleich zu Stärke aus Kartoffel-Pflanzenzellen von entsprechenden Pflanzen des waxy-Phänotyps einen erhöhten Phosphatgehalt aufweisen.

Der Amylopektingehalt kann dabei nach der in den Beispielen für Kartoffelstärke beschriebenen Methode von Hovenkamp-Hermelink et al. (Potato Research 31, (1988), 241-246) bestimmt werden. Diese Methode ist auch auf isolierte Stärken anderer Pflanzenspezies anwendbar. Verfahren zur Isolierung von Stärken sind dem Fachmann bekannt.

Der Begriff "erhöhter Phosphatgehalt" bedeutet im Zusammenhang mit der vorliegenden Erfindung, daß der Gesamtgehalt an kovalent gebundenem Phosphat und/oder der Gehalt an Phosphat in C-6-Position der in den erfindungsgemäßen Pflanzenzellen synthetisierten Stärke um mindestens 30%, bevorzugt um mindestens 50%, besonders bevorzugt um mindestens 75%, insbesondere um mindestens 100% im Vergleich zu Kartoffel-Pflanzenzellen von entsprechenden Kartoffel-Pflanzen des waxy-Phänotyps erhöht ist. Die Bestimmung des Gesamt-Phosphatgehalts bzw. des Gehalts an Phosphat in C-6-Position kann nach der unten beschriebenen Methode erfolgen.

Unter dem Begriff "entsprechende Pflanzen des waxy-Phänotyps" sollen im Zusammenhang mit der vorliegenden Erfindung vergleichbare Kartoffel-Pflanzen verstanden werden, vorzugsweise Kartoffel-Pflanzen der gleichen Ursprungssorte, d.h., der Sorte aus der die erfindungsgemäßen transgenen Kartoffel-Pflanzen durch Einführung der oben beschriebenen genetischen Modifikation hervorgegangen sind. Ferner enthalten Pflanzen des waxy-Phänotyps Kartoffel-Pflanzenzellen, die eine Stärke mit einem Amylopektingehalt von mindestens 90%, bevorzugt von mindestens 93%, besonders bevorzugt von mindestens 95% und insbesondere bevorzugt von mindestens 97% synthetisieren. Des weiteren sind Kartoffel-Pflanzen des waxy-Phänotyps dadurch gekennzeichnet, daß sie eine verringerte enzymatische Aktivität des GBSSI-Proteins im Vergleich zu Kartoffel-Pflanzenzellen von Kartoffel-Wildtyppflanzen aufweisen.

Die vorliegenden Erfindung beschreibt auch transgene Kartoffel-Pflanzenzellen, die eine modifizierte Stärke mit einem Amylopektingehalt von mindestens 90%, bevorzugt von mindestens 93%, besonders bevorzugt von mindestens 95% und insbesondere bevorzugt von mindestens 97% enthalten, im Vergleich zu Stärke aus Kartoffel-Pflanzenzellen von entsprechenden Pflanzen des waxy-Phänotyps einen erhöhten Phosphatgehalt aufweisen und/oder eine im Vergleich zu Stärken aus Kartoffel-Pflanzenzellen oder entsprechenden Kartoffel-Pflanzen des waxy-Phänotyps eine verringerte Verkleisterungstemperatur aufweisen.

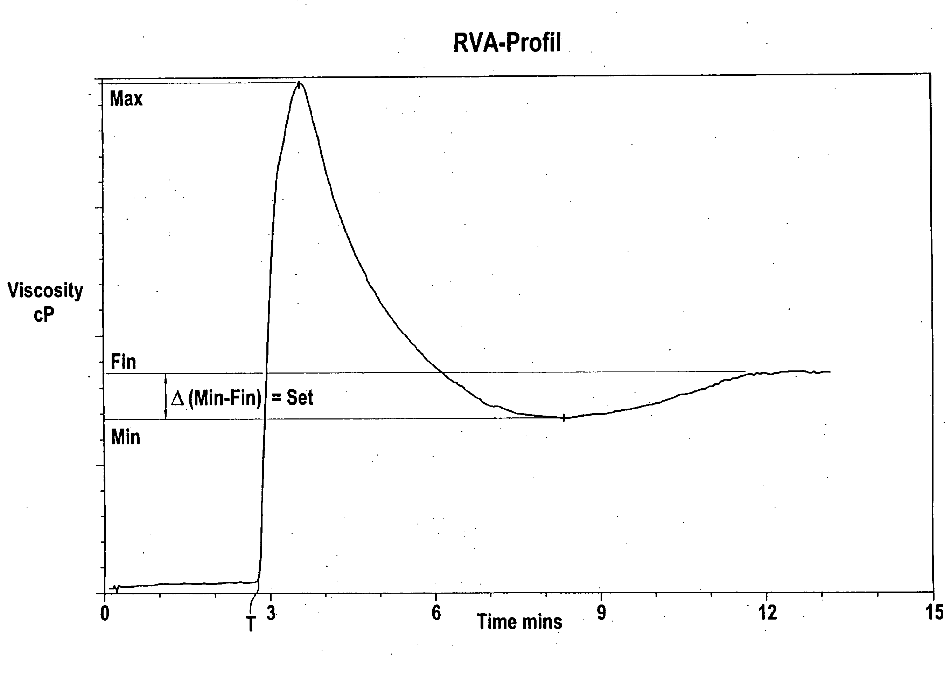

Unter der Verkleisterungstemperatur soll im Rahmen der vorliegenden Erfindung die Temperatur T verstanden werden, die sich aus einem Viskositätsprofil ermitteln läßt (s. Figur 1), das mittels eines Rapid Visco Analysers (RVA) (Newport Scientific Pty Ltd, Investment Support Group, Warriewood, NSW 2102, Australien) gewonnen werden kann. Die Erstellung des Viskositätsprofils erfolgt dabei nach dem unten beschriebenen Protokoll. Die Verkleisterungstemperatur bezeichnet diejenige Temperatur, bei der die Viskosität aufgrund der Quellung der Stärkekömer beginnt signifikant anzusteigen. Die Bestimmung der Verkleisterungstemperatur erfolgt über die Steigung der Viskositätskurve in Abhängigkeit von der Zeit. Wird die Steigung der Kurve größer als 1,2 (dieser Wert wird vom Benutzer am RVA-Gerät vorgegeben), identifiziert das Computerprogramm die zu diesem Zeitpunkt gemessene Temperatur als Verkleisterungstemperatur.

Der Begriff "verringerte Verkleisterungstemperatur" bedeutet dabei, daß die Verkleisterungstemperatur im Vergleich zu Kartoffel-Stärken aus Kartoffel-Pflanzenzellen von entsprechenden Kartoffel-Pflanzen des waxy-Phänotyps um mindestens 0.5°C, bevorzugt um mindestens 1.5°C, besonders bevorzugt um mindestens 3°C und insbesondere bevorzugt um mindestens 5°C verringert ist.

Die Beobachtung, daß die Stärken der erfindungsgemäßen Kartoffel-Pflanzenzellen eine im Vergleich zu Stärken aus Kartoffel-Pflanzenzellen von entsprechenden Kartoffel-Pflanzen des waxy-Phänotyps verringerte Verkleisterungstemperatur aufweisen, ist insbesondere deshalb überraschend, weil Safford et al. (Carbohydrate Polymeres 35, (1998), 155-168) zeigen konnten, daß ein im Vergleich zu Stärken aus Kartoffel-Wildtyppflanzen erhöhter Phosphatgehalt, der durch eine antisense-Inhibierung eines BEI-Proteins in Kartoffelpflanzen hervorgerufen werden kann, zu Kartoffel-Stärken mit einer erhöhten "viscosity onset temperature" führt. Die "viscosity onset temperature" ist eine der Verkleisterungstemperatur verwandte Größe, die im Unterschied zur Verkleisterungstemperatur auf der Methode der "Differential Scanning Calorimetry" (=DSC) beruht.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten.

Die Verwendung der Agrobakterien-vermittelten Transformation von Pflanzenzellen ist intensiv untersucht und ausreichend in EP-A-120516; Hoekema, in: The Binary Plant Vector System Offsetdrukkerij Kanters B.V., Alblasserdam (1985), Chapter V; Fraley et al., Crit. Rev. Plant Sci. 4, 1-46 und An et al. EMBO J. 4, (1985), 277-287 beschrieben worden. Für die Transformation von Kartoffel, siehe z.B. Rocha-Sosa et al., EMBO J. 8, (1989), 29-33).

Generell kommt für die Expression des fremden Nucleinsäuremoleküls (der fremden Nucleinsäuremoleküle) jeder in pflanzlichen Zellen aktive Promotor in Frage. Der Promotor kann dabei so gewählt sein, daß die Expression in den erfindungsgemäßen Pflanzen konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein.

Sinnvolle Promotoren sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451), der Ca/b-Promotor (s. beispielsweise US 5656496, US 5639952, Bansal et al., Proc. Natl. Acad. Sci.USA 89, (1992), 3654-3658) und der Rubisco SSU-Promotor (s. beispielsweise US 5034322, US 4962028) oder für eine endosperm-spezifische Expression der Glutelin-Promotor (Leisy et al., Plant Mol. Biol. 14, (1990), 41-50; Zheng et al., Plant J. 4, (1993), 357-366; Yoshihara et al., FEBS Lett. 383, (1996), 213-218), der Shrunken-1 Promotor (Werr et al., EMBO J. 4, (1985), 1373-1380), der HMG-Promotor aus Weizen, der USP-Promotor, der Phaseolinpromotor oder Promotoren von Zein-Genen aus Mais (Pedersen et al., Cell 29, (1982), 1015-1026; Quatroccio et al., Plant Mol. Biol. 15 (1990), 81-93).

Die Expression des fremden Nucleinsäuremoleküls (der fremden Nucleinsäuremoleküle) ist insbesondere in solchen Organen der Pflanze von Vorteil, die Stärke speichern. Solche Organe sind z.B. die Knolle der Kartoffelpflanze oder die Körner bzw. das Endosperm von Mais-, Weizen- oder Reispflanzen. Bevorzugt werden daher Promotoren verwendet, die die Expression in diesen Organen vermitteln.

Es können jedoch auch Promotoren verwendet werden, die nur zu einem durch äußere Einflüsse determinierten Zeitpunkt aktiviert werden (siehe beispielsweise WO 93/07279-A1). Von besonderem Interesse können hierbei Promotoren von heatshock Proteinen sein, die eine einfache Induktion erlauben. Ferner können samenspezifische Promotoren, wie z.B. der USP-Promoter aus Vicia faba, eine samen spezifische Expression in Vicia faba und anderen Pflanzen gewährleisten (Fiedler et al., Plant Mol. Biol. 22, (1993), 669-679; Bäumlein et al., Mol. Gen. Genet. 225, (1991), 459-467). Ferner können auch fruchtspezifische Promotoren eingesetzt werden, wie z.B. beschrieben in der WO 91/01373-A1.

Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z.B. Gielen et al., EMBO J. 8 (1989), 23-29) und sind beliebig austauschbar.

In einer besonders bevorzugten Ausführungsform betrifft die Erfindung Pflanzenzellen aus Kartoffel.

Die erfindungsgemäßen Kartoffel-Pflanzenzellen können zur Regeneration ganzer Kartoffel-Pflanzen verwendet werden.

Die durch Regeneration der erfindungsgemäßen transgenen Kartoffel-Pflanzenzellen erhältlichen Kartoffel-Pflanzen sind ebenfalls Gegenstand der vorliegenden Erfindung. Ferner sind Gegenstand der Erfindung Kartoffel-Pflanzen, die die oben beschriebenen transgenen Kartoffel-Pflanzenzellen enthalten. Die Erfindung betrifft Kartoffelpflanzen.

Die vorliegende Erfindung beschreibt auch ein Verfahren zur Herstellung einer transgenen Kartoffel-Pflanzenzelle oder Kartoffel-Pflanze, die eine modifizierte Stärke synthetisiert, wobei

(a) eine Kartoffel-Zelle genetisch modifiziert wird durch die Einführung eines oder mehrerer fremder Nucleinsäuremoleküle, deren Vorhandensein und/oder Expression zur Verringerung der Aktivität eines Proteins mit der Aktivität eines GBSSI-Proteins und zur Verringerung der Aktivität eines Proteins mit der Aktivität eines BE-Proteins führen; und im Fall der Herstellung einer Kartoffel-Pflanze

(b) aus der gemäß Schritt a) hergestellten Kartoffel-Zelle eine Kartoffel-Pflanze regeneriert wird; und aus der gemäß Schritt b) erzeugten Kartoffel-Pflanze gegebenenfalls weitere Kartoffel-Pflanzen erzeugt werden.

Die vorliegende Erfindung beschreibt auch ein Verfahren zur Herstellung einer transgenen Kartoffel-Pflanzenzelle oder Pflanze, deren Stärke einen Amylopektingehalt von mindestens 90% und einen erhöhten Phosphatgehalt im Vergleich zu Stärke aus entsprechenden Kartoffel-Pflanzen des waxy-Phänotyps aufweist, wobei

(a) eine Kartoffel Zelle genetisch modifiziert wird durch die Einführung eines oder mehrerer fremder Nucleinsäuremolekül(s)e, deren/dessen Vorhandensein oder deren/dessen Expression zur Verringerung der Aktivität eines Proteins mit der Aktivität eines GBSSI-Proteins und zur Verringerung der Aktivität eines Proteins mit der Aktivität eines BEI-Proteins führt/führen; und im Fall der Herstellun einen Kartoffel-Pflanze

(b) aus der gemäß Schritt a) hergestellten Kartoffel-Zelle eine Kartoffel-Pflanze regeneriert wird; und aus der gemäß Schritt b) erzeugten Kartoffel-Pflanze gegebenenfalls weitere Kartoffel-Pflanzen erzeugt werden.

Ferner beschreibt die vorliegende Erfindung ein Verfahren zur Herstellung einer transgenen Kartoffel-Pflanzenzelle oder -Pflanze, deren Stärke einen Amylopektingehalt von mindestens 90% und die einen erhöhten Phosphatgehalt und/oder eine verringerte Verkleisterungstemperatur T im Vergleich zu Stärke aus entsprechenden Kartoffel-Pflanzen des waxy-Phänotyps aufweist, wobei

(a) eine Kartoffel-Zelle genetisch modifiziert wird durch die Einführung eines oder mehrerer fremder Nucleinsäuremolekül(s)e, deren/dessen Vorhandensein oder deren/dessen Expression zur Verringerung der Aktivität eines Proteins mit der Aktivität eines GBSSI-Proteins und zur Verringerung der Aktivität eines Proteins mit der Aktivität eines BEI-Proteins führt/führen; und im Fall der Herstellung einer Kartoffel-Pflanze

(b) aus der gemäß a) hergestellten Kartoffel-Zelle eine Kartoffel-Pflanze regeneriert wird; und aus der gemäß Schritt b) erzeugten Kartoffel-Pflanze gegebenenfalls weitere Kartoffel-Pflanzen erzeugt werden.

Die Begriffe "erhöhter Phosphatgehalt" bzw "verringerte Verkleisterungstemperatur" werden in diesem Zusammenhang wie bereits oben definiert.

Für die laut Schritt a) eingeführte genetische Modifikation gilt dasselbe, was bereits oben in anderem Zusammenhang mit den erfindungsgemäßen Kartoffel-Pflanzen erläutert wurde.

Die Regeneration von Kartoffel-Pflanzen gemäß Schritt b) kann nach dem Fachmann bekannten Methoden erfolgen.

Die Erzeugung weiterer Kartoffel-Pflanzen gemäß Schritt b) der erfindungsgemäßen Verfahren kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewähle Pflanzen mit bestimmten Eigenschaften miteinander gekreuzt und vermehrt. Natürlich ist dem Fachmann bekannt, daß er zur Herstellung der erfindungsgemäßen Kartoffel-Pflanzenzellen und -Pflanzen auch transgene Kartoffel-Pflanzen verwenden kann, bei denen bereits die Aktivität eines der vorgenannten Proteine verringert ist und die gemäß dem erfindungsgemäßen Verfahren lediglich nur noch insoweit genetisch modifiziert werden müssen, daß die Aktivität des zweiten Proteins ebenfalls verringert wird.

Ferner ist dem Fachmann bekannt, daß die oben beschriebene Supertransformation nicht unbedingt bei Primärtransformanten durchgeführt wird, sondern vorzugsweise bei zuvor ausgewählten stabilen transgenen Kartoffel-Pflanzen, die vorteilhafterweise bereits durch entsprechende Experimente auf beispielsweise Fertilität, stabile Expression des Fremdgens, Hemi- und Homozygotie etc. getestet wurden.

Die vorliegende Erfindung betrifft auch die durch die erfindungsgemäßen Verfahren erhältlichen Kartoffel-Pflanzen.

Die vorliegende Erfindung betrifft auch Vermehrungsmaterial erfindungsgemäßer Kartoffel-Pflanzen sowie der gemäß den erfindungsgemäßen Verfahren hergestellten transgenen Kartoffel-Pflanzen. Der Begriff Vermehrungsmaterial umfaßt dabei jene Bestandteile der Kartoffel-Pflanze, die geeignet sind zur Erzeugung von Nachkommen auf vegetativem oder generativem Weg. Für die vegetative Vermehrung eignen sich beispielsweise Stecklinge, Calluskulturen, Rhizome oder Knollen. Anderes Vermehrungsmaterial umfaßt beispielsweise Früchte, Samen, Sämlinge, Protoplasten, Zellkulturen etc.. Vorzugsweise handelt es sich bei dem Vermehrungsmaterial um Knollen und Samen.

Ferner betrifft die vorliegende Erfindung die Verwendung von einem oder mehreren fremden Nucleinsäuremolekülen, die ein Protein mit der enzymatischen Aktivität eines GBSSI-Proteins und ein Protein mit der enzymatischen Aktivität eines BE-Proteins codieren oder die Verwendung von Fragmenten besagter Nucleinsäuremoleküle zur Herstellung von erfindungsgemäßen Kartoffel-Pflanzenzellen oder -Pflanzen, die eine modifizierte Stärke mit einem Amylopektingehalt von mindestens 90% und einer verringerten Verkleisterungstemperatur im Vergleich zu Stärke aus Kartoffel-Pflanzenzellen von entsprechenden Kartoffelpflanzen des waxy-Phänotyps synthetisieren.

In einer weiteren bevorzugten Ausführungsform betrifft die vorliegende Erfindung die Verwendung von einem oder mehreren fremden Nucleinsäuremolekül(en), das (die) ein Protein mit der enzymatischen Aktivität eines GBSSI-Proteins und ein Protein mit der enzymatischen Aktivität eines BEI-Proteins codiert (codieren) oder die Verwendung von Fragmenten besagten Nucleinsäuremoleküls/besagter Nucleinsäuremoleküle, zur Herstellung von erfindungsgemäßen Kartoffel-Pflanzen, die eine modifizierte Stärke synthetisieren, die im Vergleich zu Stärke aus entsprechenden Pflanzen des waxy-Phänotyps einen erhöhten Phosphatgehalt und/oder eine verringerte Verkleisterungstemperatur aufweist.

Unter dem Begriff "Fragment(en)" soll in diesem Zusammenhang ein Teil des fremden Nucleinsäuremoleküls bzw. der fremden Nucleinsäuremoleküle verstanden werden, der beispielsweise für einen funktionell aktiven Teil der beschriebenen Proteine codieren kann. Ferner kann das Fragment auch für eine antisense- oder eine Cosuppressions-mRNA oder für ein Ribozym codieren. Bei der Verwendung der Fragmente ist zu beachten, daß nur solche Fragmente verwendet werden dürfen, die zu einer Verringerung der enzymatischen Aktivität eines GBSSI-und/oder eines BE- bzw. eines BEI-Proteins führen.

In einer weiteren Ausführungsform beschreibt die vorliegende Erfindung die Verwendung von einem oder mehreren fremden Nucleinsäuremolekül(en) zur Herstellung von Kartoffel-Pflanzen, die eine Stärke mit einem Amylopektingehalt von mindestens 90% synthetisieren, die im Vergleich zu Stärke aus entsprechenden Kartoffel-Pflanzen des waxy-Phänotyps einen erhöhten Phosphatgehalt und/oder eine verringerte Verkleisterungstemperatur aufweist, wobei das fremde Nucleinsäuremolekül ein Molekül ist oder die fremden Nucleinsäuremoleküle mehrere Moleküle sind, ausgewählt aus der Gruppe bestehend aus:

a) DNA-Molekülen, die mindestens eine antisense-RNA codieren, die eine Verringerung der Expression von endogenen GBSSI- und/oder BEI-Proteinen codierenden Genen bewirken;
b) DNA-Molekülen, die über einen Cosuppressionseffekt zu einer Verringerung der Expression von endogenen Genen, die GBSSI- und/oder BEI-Proteine codieren, führen;
c) DNA-Moleküle, die mindestens ein Ribozym codieren, das spezifisch Transkripte von endogenen Genen spaltet, die GBSSI- und/oder BEI-Proteine codieren; und
d) Mittels in vivo-Mutagenese eingeführte Nucleinsäuremoleküle, die zu einer Mutation oder einer Insertion einer heterologen Sequenz in einem oder mehreren endogenen GBSSI- und/oder BEI-Proteine codierenden Gen führen, wobei die Mutation oder Insertion eine Verringerung der Expression des GBSSI-Gens und/oder des BEI-Gens bewirkt, oder die Synthese eines inaktiven GBSSI-Proteins und/oder eines inaktiven BEI-Proteins.

Wie vorstehend bereits erläutert, können die fremden Nudeinsäuremoteküle zeitgleich oder auch nacheinander in das Genom der Kartoffel-Pflanzenzelle eingeführt werden. Zeit- und kostensparender ist dabei die zeitgleiche Einführung der fremden Nucleinsäuemoleküle, d.h. die Cotransformation bei der vorzugsweise in einem Transformationsexperiment gemäß der oben beschriebenen erfindungsgemäßen Verfahren (ein) Nucleinsäuremolekül(e) in die Kartoffel-Pflanzenzelle eingeführt wird, deren/dessen Vorhandensein und gegebenenfalls Expression zur Verringerung der Aktivität eines Proteins mit der Aktivität eines GBSSI-Proteins und zur Verringerung der Aktivität eines Proteins mit der Aktivität eines BE-Proteins, vorzugsweise eines BEI-Proteins, führt/führen. Daher beschreibt die vorliegende Erfindung auch Zusammensetzungen, die mindestens eines der vorstehend beschriebenen Nucleinsäuremoleküle enthalten. Vorzugsweise führt deren Einführung in Pflanzenzellen zur Verringerung der Aktivität eines GBSSI-Proteins und zur Verringerung der Aktivität eines Proteins mit der Aktivität eines BE-Proteins, vorzugsweise eines BEI-Proteins. Dabei können in der Zusammensetzung die Nucleinsäuremoleküle, deren jeweiliges Vorhandensein in der Kartoffel-Pflanzenzelle zur Verringerung der Aktivitäten von GBSSI- und der BE-Proteinen, vorzugsweise eines BEI-Proteins, führen, entweder getrennt oder zusammen in einem rekombinanten Nucleinsäuremolekül umfaßt sein. Im ersten Fall, kann die Zusammensetzung beispielsweise zwei oder mehrere rekombinante Vektoren enthalten, deren gemeinsames Vorhandensein in der Pflanzenzelle zu dem besagten Phänotyp führt. Im zweiten Falle, der bevorzugt wird, enthält ein rekombinantes Nucleinsäuremolekül die genetische Information, die zur Verringerung der Aktivität eines GBSSI- und eines BE-Proteins, vorzugsweise eines BEI-Proteins, führt. Beispielsweise können in einem solchen rekombinanten Nucleinsäuremolekül die vorstehend beschriebenen Nucleinsäuremoleküle, deren Vorhandensein in einer Pflanzenzelle zur Verringerung der Aktivität eines GBSSI- bzw. eines BE- oder BEI-Proteins führt, als ein chimäres Gen oder als getrennte Gene vorliegen. Beispiele für solche Doppel- oder Mehrfachkonstrukte sind in der Fachliteratur zahlreich beschrieben. Die vorgenannten rekombinanten Nucleinsäuremoleküle können in jeder beliebigen Wirtszelle vorliegen, Ein weiterer Vorteil bei der Verwendung solcher Doppel- oder Mehrfachkonstrukte ist, daß erfindungsgemäße Kartoffel-Pflanzenzellen und -Pflanzen leichter identifiziert werden können, beispielsweise durch eine entsprechende Auswahl von PCR-Primem oder Southem blots. Daher sind die erfindungsgemäßen Kartoffel-Pflanzenzellen und -Pflanzen vorzugsweise durch das Vorhandensein solcher Doppel- oder Mehrfachkonstrukte gekennzeichnet.

Die beschriebenen transgenen Kartoffel-Pflanzenzellen und -Pflanzen synthetisieren aufgrund der Expression eines fremden Nucleinsäuremoleküls oder mehrerer fremder Nucleinsäuremoleküle, dessen/deren Vorhandensein oder dessen/deren Expression zur Verringerung der Aktivität eines GBSSI-Proteins und zur Verringerung der Aktivität eines BE-Proteins, vorzugsweise des BEI-Proteins, führt/führen im Vergleich zu entsprechenden nicht genetisch modifizierten Kartoffel-Pflanzenzellen von Kartoffel-Wildtyppflanzen, eine Stärke, die in ihren physikalisch-chemischen Eigenschaften, insbesondere dem Amylose/Amylopektin-Verhältnis und/oder dem Phosphatgehalt und/oder dem Verkleisterungsverhalten im Vergleich zu in Kartoffel-Wildtyp-Pflanzen synthetisierter Stärke verändert ist.

Die vorliegende Erfindung beschreibt daher Stärke, die aus den erfindungsgemäßen transgenen Kartoffel-Pflanzenzellen, -Pflanzen sowie Vermehrungsmaterial erhältlich ist.

Ferner beschreibt die vorliegende Erfindung auch Kartoffel-Stärken, die dadurch gekennzeichnet sind, daß sie einen Amylopektingehalt von mindestens 90% und einen im Vergleich zu Kartofell-Stärke aus entsprechenden Kartofell-Pflanzen des waxy-Phänotyps um mindestens 30%, bevorzugt um mindestens 50 %, besonders bevorzugt um mindestens 75%, insbesondere um mindestens 100% erhöhten Phosphatgehalt im Vergleich zu Kartoffel-Pflanzenzellen von entsprechenden Pflanzen des waxy-Phänotyps aufweisen.

Aufgrund des erhöhten Phosphatgehaltes weisen die beschriebenen Kartoffel-Stärken gegenüber herkömmlichen waxy-Stärken den Vorteil auf, daß sie aufgrund ihrer veränderten physiko-chemischen Eigenschaften für bestimmte Verwendungszwecke besser geeignet sind. Aufgrund des erhöhten Phosphatgehalts müssen diese Stärken im Vergleich zu herkömmlichen waxy-Stärken hierfür nicht oder weniger stark nachträglich chemisch phosphoryliert werden.

Vorzugsweise unterscheiden sich die beschriebenen Kartoffel-Stärken von chemisch phosphorylierten Monophosphat-waxy-Stärken in ihren strukturellen/funktionellen Eigenschaften.

Phosphorylierte waxy-Stärken eignen sich besonders zur Verwendung für alle Dickungen ohne Haut bzw. Gelbildung im Dessert, Feinkost- und Fertiggerichtberich vor allem auch für Tiefkühlerzeugnisse. In technischen Bereichen werden Monostärkephosphate teilweise zur Papierherstellung, außerdem als Schlichte-, Flockungs- und Flotationsmittel sowie als Waschmittelzusatz verwendet.

Die vorliegenden Erfindung beschreibt auch Kartoffel-Stärken, die dadurch gekennzeichnet sind, daß sie einen Amylopektingehalt von mindestens 90%, einen im Vergleich zu Kartoffel-Stärke aus entsprechenden Kartoffel-Pflanzen des waxy-Phänotyps um mindestens 30% erhöhten Phosphatgehalt und/oder eine verringerte Verkleisterungstemperatur T aufweisen.

Der Begriff "verringerte Verkleisterungstemperatur" soll in diesem Zusammenhang wie bereits oben definiert verstanden werden.

Im Zusammenhang mit bestimmten Anwendungen und technischen Prozessen erlaubt eine verringerte Verkleisterungstemperatur die Einsparung von Wärmeenergie und/oder eine vereinfachte Prozeßführung.

Die vorliegende Erfindung beschreibt Stärken aus Kartoffelpflanzen, die dadurch gekennzeichnet sind, daß sie einen Amylopektingehalt von mindestens 90%, einen im Vergleich zu Kartoffel-Stärke aus entsprechenden Kartoffel-Pflanzen des waxy-Phänotyps um mindestens 30% erhöhten Phosphatgehalt und/oder eine verringerte Verkleisterungstemperatur T aufweisen.

Native Kartoffelstärken zeigen im Vergleich zu nativen Stärken von Mais, Reis- oder Weizenpflanzen einen erhöhten Phosphatgehalt, was sie für bestimmte Anwendungen prädestiniert. Überraschenderweise ist es mit Hilfe des erfindungsgemäßen Ansatzes möglich, den Phosphatgehalt von waxy-Kartoffelstärken weiter zu steigern, so daß diese Kartoffelstärken im Vergleich zu Kartoffelstärken von entsprechenden Wildtyppflanzen und/oder im Vergleich zu entsprechenden Kartoffelpflanzen des waxy-Phänotyps einen um mindestens 30%, bevorzugt um mindestens 50%, besonders bevorzugt um mindestens 75% und insbesondere von mindestens 100% erhöhten Phosphatgehalt aufweisen. Ferner besitzen Kartoffelstärken gegenüber Cerealienstärken (z.B. Weizen, Hafer, Mais, Reis) den Vorteil, daß sie einen geringen Gehalt an Lipiden und Proteinen aufweisen.

Die Erfindung beschreibt Kartoffelstärken, die gekennzeichnet sind durch einen Amylopektingehalt von mindestens 93%, besonders bevorzugt von mindestens 95% und insbesondere bevorzugt von mindestens 97% und/oder durch einen im Vergleich zu Kartoffel-Starke aus Kartoffel-Pflanzenzellen von entsprechenden Kartoffel-Pflanzen des waxy-Phänotyps um mindestens 30%, bevorzugt um mindestens 50%, besonders bevorzugt um mindestens 75%, insbesondere um mindestens 100% erhöhten Phosphatgehalt im Vergleich zu Kartoffel-Pflanzenzellen von entsprechenden Kartoffel-Pflanzen des waxy-Phänotyps und/oder durch eine im Vergleich zu Stärke aus Kartoffel-Pflanzenzellen von entsprechenden Kartoffel-Pflanzen des waxy-Phänotyps um mindestens 0.5°C, bevorzugt um mindestens 1.5°C, besonders bevorzugt um mindestens 3°C und insbesondere bevorzugt um mindestens 5°C verringerte Verkleisterungstemperatur.

Die Begriffe "Phosphatgehalt" und "Verkleisterungstemperatur" werden in diesem Zusammenhang in gleicher Weise wie bereits oben beschrieben definiert.

Die vorliegende Erfindung betrifft ferner ein Verfahren zur Herstellung einer modifizierten Stärke umfassend den Schritt der Extraktion der Stärke aus einer oben beschriebenen erfindungsgemäßen Kartoffel-Pflanze(nZelle) und/oder aus stärkespeichernden Teilen einer solchen Kartoffel-Pflanze. Vorzugsweise umfaßt ein solches Verfahren auch den Schritt des Erntens der kultivierten Kartoffel-Pflanzen und/oder stärkespeichernder Teile dieser Kartoffel-Pflanzen vor der Extraktion der Stärke und besonders bevorzugt ferner den Schritt der Kultivierung erfindungsgemäßer Kartoffel-Pflanzen vor dem Ernten. Verfahren zur Extraktion der Stärke von Pflanzen oder von stärkespeichemden Teilen von Pflanzen sind dem Fachmann bekannt. Weiterhin sind Verfahren zur Extraktion der Stärke aus verschiedenen anderen stärkespeichemden Pflanzen beschrieben, z. B. in "Starch: Chemistry and Technology (Hrsg.: Whistler, BeMiller und Paschall (1994), 2. Ausgabe, Academic Press Inc. London Ltd; ISBN 0-12-746270-8; siehe z. B. Kapitel XII, Seite 412-468: Mais und Sorghum-Stärken: Herstellung; von Watson; Kapitel XIII, Seite 469-479: Tapioca-, Arrowroot- und Sagostärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996) 54-57, die Extraktion von Maisstärke im industriellen Maßstab wird in der Regel durch das sogenannte "wet milling" erreicht.)). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

Die durch das erfindungsgemäße Verfahren zur Verfügung gestellten Kartoffel-Stärken können nach dem Fachmann bekannten Verfahren nachträglich modifiziert werden und eignen sich in unmodifizierter oder modifizierter Form für verschiedene Verwendungen im Nahrungsmittel- oder Nicht-Nahrungsmittelbereich.

Grundsätzlich läßt sich die Einsatzmöglichkeit der Stärke in zwei große Bereiche unterteilen. Der eine Bereich umfaßt die Hydrolyseprodukte der Stärke, hauptsächlich Glucose und Glucanbausteine, die über enzymatische oder chemische Verfahren erhalten werden. Sie dienen als Ausgangsstoffe für weitere chemische Modifikationen und Prozesse, wie Fermentation. Für eine Reduktion der Kosten kann hierbei die Einfachheit und kostengünstige Ausführung eines Hydrolyseverfahrens von Bedeutung sein. Gegenwärtig verläuft es im wesentlichen enzymatisch unter Verwendung von Amyloglucosidase. Vorstellbar wäre eine Kosteneinsparung durch einen geringeren Einsatz von Enzymen. Eine Strukturveränderung der Stärke, z.B. Oberflächenvergrößerung des Korns, leichtere Verdaulichkeit durch geringeren Verzweigungsgrad oder eine sterische Struktur, die die Zugänglichkeit für die eingesetzten Enzyme begrenzt, könnte dies bewirken.

Der andere Bereich, in dem die Stärke wegen ihrer polymeren Struktur als sogenannte native Stärke verwendet wird, gliedert sich in zwei weitere Einsatzgebiete:

1. Nahrungsmittelindustrie
Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z.B. anorganischen oder organischen Ionen.

2. Nicht-Nahnrngmittelindustrie
In diesem großen Bereich kann die Stärke als Hilfsstoff für unterschiedliche Herstellungsprozesse bzw. als Zusatzstoff in technischen Produkten eingesetzt. Bei der Verwendung der Stärke als Hilfsstoff ist hier insbesondere die Papier- und Pappeindustrie zu nennen. Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.

2.1 Papier- und Pappeindustrie
Innerhalb des Papierherstellungsprozesses sind vier Anwendungsbereiche, nämlich Oberfläche, Strich, Masse und Sprühen, zu unterscheiden. Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papiervlies von Bedeutung. Beim Einsatz der Stärke im Sprühbereich sind ebenfalls ein angepaßter Feststoffgehalt, hohe Viskosität sowie ein hohes Bindevermögen von Bedeutung.

2.2 Klebstoffindustrie
Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.

2.3 Textil- und Textilpflegemittelindustrie
Ein großes Einsatzfeld für die Stärken als Hilfsmittel und Zusatzstoffe ist der Bereich Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechternden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.

2.4 Baustoffindustrie
Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte zur Verzögerung der Abbindung eingesetzt.

2.5 Bodenstabilisation
Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und verkrustungsmindemden Wirkung den bisher eingesetzten Produkten gleichzusetzen, liegen preislich aber deutlich unter diesen.

2.6 Einsatz bei Pflanzenschutz- und Düngemitteln
Ein Einsatzbereich liegt bei der Verwendung der Stärke in Pflanzenschutzmitteln zur Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.

2.7 Pharmaka, Medizin und Kosmetikindustrie
Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbiert und nach kurzer Zeit soweit quellt, daß der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.

2.8 Stärkezusatz zu Kohlen und Briketts

Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.

2.9 Erz- und Kohleschlammaufbereitung
Die Stärke kann ferner bei der Erz- und Kohleschlammaufbereitung als Flockungsmittel eingesetzt werden.

2.10 Gießereihilfsstoff
Ein weiterer Einsatzbereich besteht als Zusatz zu Gießereihilfsstoffen. Bei verschiedenen Gußverfahren werden Kerne benötigt, die aus Bindemittelversetzten Sänden hergestellt werden. Als Bindemittel wird heute überwiegend Bentonit eingesetzt, das mit modifizierten Stärken, meist Quellstärken, versetzt ist.
Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.

2.11 Einsatz in der Kautschukindustrie
In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.

2.12 Herstellung von Lederersatzstoffen
Eine weitere Absatzmöglichkeit der modifizierten Stärken besteht bei der Herstellung von Lederersatzstoffen.

2.13 Stärke in synthetischen Polymeren
Auf dem Kunststoffsektor zeichnen sich folgende Einsatzgebiete ab: die Einbindung von Stärkefolgeprodukten in den Verarbeitungsprozeß (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyethylen. Hierbei werden die Stärke und das synthetische Polymer durch Koexpression im Verhältnis von 1 : 1 zu einem 'master batch' kombiniert, aus dem mit granuliertem Polyethylen unter Anwendung herkömmlicher Verfahrenstechniken diverse Produkte hergestellt werden. Durch die Einbindung von Stärke in Polyethylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wäßrigen Farben erreicht werden.
Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.
Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des weiteren sind Stärke/Polymermischungen günstig zu beurteilen, da sie eine sehr viel höhere biologische Abbaubarkeit aufweisen.
Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten wie Windeln und Unterlagen sowie im landwirtschaftlichen Sektor, z.B. bei Saatgutpillierungen.

Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und -form sowie Kristallinität, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur und -transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.

Die Erzeugung modifizierter Stärken mittels gentechnischer Eingriffe in einer transgenen Pflanze kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderte Stärken weiteren chemischen und/oder physikalischen Modifikationen unterworfen werden, was zu weiteren Verbesserungen der Qualität für bestimmte der oben beschriebenen Einsatzgebiete führen. Diese chemischen und physikalischen Modifikationen sind grundsätzlich bekannt. Insbesondere handelt es sich dabei um Modifikationen durch:

- Hitzebehandlung,
- Säurebehandlung,
- Erzeugung von Stärkeethern
  Stärke-Alkylether, O-Allylether, Hydroxylalkylether,
  O-Carboxylmethylether, N-haltige Stärkeether, P-haltige Stärkeether,
  S-haltige Stärkeether
- Erzeugung von vernetzten Stärken
- Erzeugung von Stärke-Pfropf-Polymerisaten
- Oxidation und
- Veresterungen, welche zur Entstehung von Phosphat-, Nitrat-, Sulfat-, Xanthat-, Acetat- und Citratstärken führen. Weitere organische Säuren können ebenfalls zur Veresterung eingesetzt werden.

Die Figuren zeigen:

Figur 1: Schematische Darstellung eines RVA-Profils

In den Beispielen wurden die folgenden Methoden verwendet:

1. Stärkeanalytik

a) Bestimmung des Amylose/Amylopektinverhältnisses Stärke wurde nach Standardmethoden aus Kartoffelpflanzen isoliert, und das Verhältnis Amylose zu Amylopektin wurde nach der von Hovenkamp-Hermelink et al. beschriebenen Methode (Potato Research 31, (1988), 241-246) bestimmt.

b) Bestimmung des Phosphatgehaltes In der Stärke können die Positionen C2, C3 und C6 der Glukoseeinheiten phosphoryliert sein. Zur Bestimmung des Phoshatgruppengehaltes an C6-Position wurden 100 mg Stärke in 1ml 0.7 M HCl für 4 Stunden bei 95°C hydrolysiert (Nielsen et al., Plant Physiol. 105, (1994), 11-117). Nach Neutralisation mit 0.7 M KOH wurden zur Glukose-6-Phosphat-Bestimmung 50 $\mu$l des Hydrolysats einem optisch-enzymatischen Test unterzogen. Die Änderung der Absorption des Testansatzes (100 mM Imidazol/HCl; 10 mM $MgCl_2$; 0.4 mM NAD; 2 units Glukose-6-phosphat-dehydrogenase aus Leuconostoc mesenteroides; 30°C) wurde bei 334 nm verfolgt.

Die Bestimmung des Gesamtphosphatgehaltes erfolgte nach der Methode von Ames (Methods in Enzymology VIII, (1966), 115-118).

Es werden ca. 50 mg Stärke mit 30 $\mu$l ethanolischer Magnesiumnitrat-Lösung versetzt und drei Stunden bei 500°C im Muffelofen verascht. Der Rückstand wird mit 300 $\mu$l 0,5 M Salzsäure versetzt und 30 min bei 60°C inkubiert. Anschließend wird ein Aliquot auf 300 $\mu$l mit 0,5 M Salzsäure aufgefüllt, zu einer Mischung aus 100 $\mu$l 10%iger Ascorbinsäure und 600 $\mu$l 0,42% Ammoniummolybdat in 2 M Schwefelsäure gegeben und 20 min bei 45°C inkubiert.

Es folgt eine photometrische Bestimmung bei 820nm unter Berücksichtigung einer Phosphat-Eichreihe als Standard.

c) Bestimmung der Gelfestigkeit (Texture Analyser)

2 g Stärke (TS) werden in 25 ml $H_2O$ verkleistert (vgl. RVA) und anschließend für 24 h luftdicht verschlossen bei 25°C gelagert. Die Proben werden unter der Sonde (runder Stempel) eines Texture Analysers TA-XT2 der Firma Stable Micro Systems fixiert und die Gelfestigkeit mit folgenden Parametern bestimmt:

- Test-Geschwindigkeit 0,5 mm/s
- Eindringtiefe 7 mm
- Kontaktfläche 113 mm$^2$
- Druck 2g

d) Viskositätsprofil

2 g Stärke (TS) werden in 25 ml H$_2$O aufgenommen und für die Analyse in einem Rapid Visco Analyser (Newport Scientific Pty Ltd., Investmet Support Group, Warriewood NSW 2102, Australien) verwendet. Der Betrieb des Gerätes erfolgt nach den Angaben des Herstellers. Zur Bestimmung der Viskosität der wäßrigen Lösung der Stärke wird die Stärkesuspension zunächst von 50°C auf 95°C erhitzt, mit einer Geschwindigkeit von 12°C pro Minute. Anschließend wird die Temperatur für 2,5 Min bei 95°C gehalten. Danach wird die Lösung von 95°C auf 50°C abgekühlt, mit einer Geschwindigkeit von 12°C pro Minute. Während der gesamten Dauer wird die Viskosität bestimmt.

Die Bestimmung der Verkleisterungstemperatur erfolgt über die Steigung der Viskositätskurve in Abhängigkeit von der Zeit. Wird die Steigung der Kurve größer als 1,2 (dieser Wert wird vom Benutzer vorgegeben), identifiziert das Computerprogramm die zu diesem Zeitpunkt gemessene Temperatur als Verkleisterungstemperatur.

e) Bestimmung von Glukose, Fruktose und Saccharose

Die Bestimmung des Gehaltes von Glukose, Fruktose und Saccharose erfolgt nach der von Stitt et al. (Methods in Enzymology 174, (1989), 518-552) beschriebenen Methode.

f) Analyse der Seitenkettenverteilung des Amylopektins

Die Seitenkettenverteilung bzw. Länge wird bestimmt wie in Lloyd et al., Biochem. J. 338, (1999), 515-521 beschrieben. Es werden folgende Elutionsbedingungen gewählt:

| Zeit | 0,15 M NaOH | 1 M NaAc in 0,15M NaOH |
| min | % | % |
| --- | --- | --- |
| 0 | 100 | 0 |
| 5 | 100 | 0 |
| 20 | 85 | 15 |
| 35 | 70 | 30 |
| 45 | 68 | 32 |
| 60 | 0 | 100 |
| 70 | 0 | 100 |
| 72 | 100 | 0 |
| 80 | 100 | 0 |

g) Korngrößenbestimmung

Die Korngrößenbestimmung wurde mit einem Fotosedimentometer des Typs "Lumosed" der Firma Retsch GmbH, Deutschland durchgeführt. Die Körngrößenverteilung wurde in wäßriger Lösung bestimmt und erfolgte nach Herstellerangaben sowie basierend auf der Literatur von z.B. H. Pitsch, Korngrößenbestimmung; LABO-1988/3 Fachzeitschrift für Labortechnik, Darmstadt

h) Wasserbindevermögen

Zur Bestimmung des Wasserbindevermögens wurde der Rückstand nach der Abtrennung des löslichen Anteils durch Zentrifugation der bei 70°C gequollenen Stärke gewogen. Das Wasserbindevermögen (WBV) der Stärke wurde auf die um die lösliche Masse korrigierte Stärkeeinwaage bezogen.

$$\text{WBV (g/g)} = (\text{Rückstand} - (\text{Einwaage} - \text{löslicher Anteil}))/(\text{Einwaage} - \text{löslicher Anteil})$$

Anteil)In den Beispielen wurden folgende Vektoren verwendet:

Angaben zum Vektor pBinAR-Hyg

Das Plasmid pBinAR ist ein Derivat des binären Vektorplasmids pBin19 (Bevan, 1984), das folgendermaßen konstruiert wurde:

ein 529 bp langes Fragment, das die Nukleotide 6909-7437 des 35S-Promotor des Blumenkohl-Mosaik-Virus umfaßt, wurde als *EcoR* I/*kpn* I-Fragment aus dem Plasmid pDH51 (Pietrzak et al, 1986) isoliert und zwischen die EcoR I- und *Kpn* I-Schnittstellen des Polylinkers von pUC18 ligiert. Dabei entstand das Plasmid pUC18-35S. Aus dem Plasmid pAGV40 (Herrera-Estrella et al, 1983) wurde mit Hilfe der Restriktionsendonucleasen *Hind* III und *Pvu* II ein 192 bp langes Fragment isoliert, das das Polyadenylierungssignal (3'-Ende) des *Octopin Synthase-Gens* (Gen 3) der T-DNA des Ti-Plasmids pTiACH5 (Gielen et al, 1984) umfaßt (Nukleotide 11749-11939). Nach Addition von Sph I-Linkern an die *Pvu* II-Schnittstelle wurde das Fragment zwischen die *Sph* I- und *Hind* III-Schnittstellen von pUC18-35S ligiert. Daraus resultierte das Plasmid pA7. Ausgehend vom Plasmid pA7 wurde das *Eco* RI-*Hind* III Fragment enthaltend den 35S RNA-Promotor, den ocs-Terminator sowie den zwischen 35S RNA-Promotor und ocs-Element gelegenen Teil des Polylinkers in das entsprechend geschnittene pBIB-Hyg-Plasmid (Becker, 1990) ligiert.

Die folgenden Beispiele erläutern die Erfindung ohne sie in irgendeiner Weise zu beschränken.

Beispiel 1: Herstellung transgener Kartoffelpflanzen, die eine verringerte Aktivität eines GBSSI- und eines BEI-Proteins aufweisen

Zur Erzeugung transgener Pflanzen, die eine verringerte Aktivität eines GBSSI- und eines BEI-Proteins aufweisen, wurden zunächst transgene Pflanzen erzeugt, die eine verringerte GBSSI-Aktivität aufwiesen. Zu diesem Zwecke wurde die T-DNA des Plasmids pB33aGBSSI-Kan mit Hilfe von Agrobakterien, wie bei Roch-Sosa et al. (EMBO J. 8, (1989), 23-29) beschrieben, in Kartoffelpflanzen transferiert.

Zur Konstruktion des Plasmides pB33aGBSSI-Kan wurde das D*ral*/*Dra*l Fragment aus der Promotorregion des Patatin Klasse I Gens B33 von *Solanum tuberosum,* umfassend die Nukleotide -1512 bis +14 (Rocha-Sosa et al., (1989), s.o.) wurde in die *Sma*l Schnittstelle des Plasmids pUC19 (Genbank Acc. No. M77789) ligiert. Aus dem entstandenen Plasmid wurde das Promotorfragment als *Eco*RI/*Hind*III Fragment in die *polylinker* Region des Plasmids pBin19 (Bevan et al., Nucl Acids Res 11, (1983), 369-385) ligiert. Anschließend wurde das 3'EcoRI Fragment, Nukleotide +1181 bis +2511 des GBSSI-Gens aus *Solanum tuberosum* (Hergersberg, Molekulare Analyse des waxy Gens aus Solanum tuberosum und Expression von waxy antisense RNA in Kartoffeln. Dissertation Universität zu Köln (1988)) in die *Eco*RI Schnittstelle des entstandenen Plasmids ligiert. Es resultierte das Plasmid pB33aGBSSI-Kan.

Nach der Transformation wurden verschiedene Linien transgener Kartoffelpflanzen identifiziert, die einen deutlich verringerten Gehalt der mRNA einer GBSSI und einer verringerten Aktivität des GBSSI-Proteins aufwiesen. Ferner synthetisieren derartige Pflanzen eine Stärke mit einem Amylopektingehalt von mindestens 90%.

Anschließend wurden zwei unabhängige Linien dieser Amylopektinsynthetisierenden Pflanzen mit dem Plasmid p35SaBEI-Hyg transformiert. Zur Konstruktion dieses Plasmides wurde ein ca. 3000 bp langes SmaI/HindIII-Fragment, enthaltend eine partielle cDNA für das BEI-Enzym aus Kartoffel (Kossmann, Klonierung und funktionelle Analyse von Genen codierend für am Kohlenhydratstoffwechsel der Kartoffel beteiligte Proteine, Dissertation Technische Universität Berlin, (1992)) geglättet und in "anti-sense"-Orientierung bezüglich des 35S-Promotors in die Smal-Schnittstelle des Vektors pBinAR-Hyg (s.o.) eingeführt.

Nach der Supertransformation wurden verschiedene unabhängige Linien identifiziert, die zwar die gleichen T-DNAs, aber an unterschiedlichen Orten im Genom integriert, enthalten, die sowohl eine verringerte GBSSI-Aktivität als auch eine signifikant verringerte Menge an BEI-mRNA aufwiesen. Es wurden Pflanzen selektiert, die im Vergleich zu entsprechenden Wildtyppflanzen eine um vorzugsweise mindestens 95% reduzierte Enzymaktivität des GBSSI-Proteins und eine um mindestens 90% reduzierte Enzymaktivität des BEI-Proteins aufweisen. Die Stärken dieser Pflanzen wurden anschließend analysiert.

Beispiel 2: Analyse der Stärke von Pflanzen mit verringerter GBSSI- und BEI-Aktivität

Die von den gemäß Beispiel 1 hergestellten transgenen Kartoffelpflanzen gebildete Stärke unterscheidet sich z.B. von in Wildtyppflanzen synthetisierter Stärke in ihrem Phosphat- oder Amylosegehalt und in den mittels RVA bestimmten Viskositäts- und Verkleisterungseigenschaften. Die Ergebnisse der physico-chemischen Charakterisierung der modifizierten Stärken sind in Tabelle 1 (Tab.1) dargestellt.

Tabelle 1:

| Nr. | Genotyp | Phosphat in C6 (%) | Gesamt-phosphat (%) | Amylose (%) | RVA Max (%) | RVA Min (%) | RVA Fin (%) | RVA Set (%) | RVAT (%) | Gelfestigkeit (%) |
|-----|---------|---------|---------|---------|---------|---------|---------|---------|---------|---------|
| 1 | Desiree (Wild-typ) | 100 | 100 | 22 | 100 | 100 | 100 | 100 | 100 | 100 |
| 2 | AsGBSS I | 110 | 119 | <4 | 70 | 90 | 84 | 57 | 104 | 21 |
| 3 | AsBEI | | 170 | 20 | 124 | 94 | 90 | 76 | 100 | 91 |
| 4 | AsGBSS I-asBEI | 181 | 189 | <4 | 69 | 84 | 78 | 51 | 102 | 21 |

Legende:
GBSSI = granule bound starch synthasel
BEI = branching enzyme I
as = antisense
RVA = Rapid Visco Analyser
Max = maximale Viskosität
Min = minimale Viskosität
Fin = Viskosität am Ende der Messung
Set = Setback = Differenz aus Min und Fin
T = Verkleisterungstemperatur

Die %-Werte sind mit Ausnahme des Amylosegehaltes auf den Wildtyp (=100%) gezogen.

**Patentansprüche**

1. Transgene Kartoffel-Pflanzenzelle, die genetisch modifiziert ist, wobei die genetische Modifikation im Vorhandensein eines oder mehrerer fremder Nucleinsäuremoleküle besteht, deren Expression zur Verringerung der Aktivität von GBSSI- und BE-Proteinen führt, im Vergleich zu entsprechenden nicht genetisch modifizierten Kartoffel-Pflanzenzellen von Wildtyppflanzen, wobei die transgene Kartoffel-Pflanzenzelle eine modifizierte Stärke mit einem Amylopektingehalt von mindestens 90% enthält und im Vergleich zu Stärke aus Kartoffel-Pflanzenzellen von entsprechenden Kartoffelpflanzen des waxy-Phänotyps eine um mindestens 0.5°C verringerte Verkleisterungstemperatur aufweist und worin besagte fremde Nucleinsäuremoleküle rekombinante Nukleinsäuremoleküle sind, die aus verschiedenen Elementen bestehen, deren Kombination oder spezifische räumliche Anordnung natürlicherweise in Kartoffelzellen nicht auftritt, und worin besagte Nukleinsäuremoleküle ausgewählt sind aus der Gruppe bestehend aus

   a) DNA-Molekülen, die mindestens eine antisense-RNA codieren, die eine Verringerung der Expression von endogenen GBSSI- und/oder BE-Proteinen codierenden Genen bewirkt, wobei die DNA-Moleküle entweder die gesamte oder Teile der für ein GBSSI-Protein und/oder ein BE-Protein codierenden Sequenz umfassen;
   b) DNA-Molekülen, die über einen Cosuppressionseffekt zu Verringerung der Expression von endogenen Genen führen, die GBSSI- und/oder BE-Proteine codieren, wobei die DNA-Moleküle entweder die gesamte oder Teile der für ein GBSSI-Protein und/oder ein BE-Protein codierenden Sequenz umfassen;
   c) DNA-Moleküle, die mindestens ein Ribozym codieren, das spezifisch Transkripte von endogenen Genen spaltet, die GBSSI- und/oder BE-Proteine codieren; und
   d) Mittels in vivo-Mutagenese eingeführte Nucleinsäuremoleküle, die zu einer Mutation oder einer Insertion einer heterologen Sequenz in endogenen GBSSI- und/oder BE-Protein codierenden Genen führen,

   wobei die Mutation oder Insertion eine Verringerung der Expression von GBSSI- und/oder BE-Genen bewirkt, oder die Synthese von inaktiven GBSSI- und/oder BE-Proteinen.

2. Transgene Kartoffel-Pflanzenzelle nach Anspruch 1, worin das Vorhandensein und/oder die Expression eines oder mehrerer fremder Nucleinsäuremoleküle zur Inhibierung der Expression von endogenen Genen führt, die GBSSI- und BE-Proteine codieren.

3. Transgene Kartoffel-Pflanzenzelle nach einem oder mehreren der Ansprüche 1 bis 2, wobei besagtes BE-Protein ein BEI-Protein und/oder das BE-Gen ein BEI-Gen ist.

4. Transgene Kartoffel-Pflanzenzelle nach einem oder mehreren der Ansprüche 1 bis 3, worin die Verkleisterungstemperatur um mindestens 1,5°C verringert ist im Vergleich zu Stärke aus Kartoffel-Pflanzenzellen von entsprechenden Kartoffelpflanzen des waxy-Phänotyps.

5. Transgene Kartoffelpflanze enthaltend Kartoffel-Pflanzenzellen nach einem oder mehreren der Ansprüche 1 bis 4.

6. Verfahren zur Herstellung einer transgenen Kartoffelpflanze, deren Stärke einen Amylopektingehalt von mindestens 90% und im Vergleich zu Stärke aus Kartoffelpflanzen von entsprechenden Kartoffelpflanzen des waxy-Phänotyps eine um mindestens 0.5°C verringerte Verkleisterungstemperatur aufweist, worin

a) eine Kartoffel-Pflanzenzelle genetisch modifiziert wird durch die Einführung eines oder mehrerer fremder Nucleinsäuremoleküle wie in Anspruch 1 definiert, deren Vorhandensein und/oder Expression zur Verringerung der Aktivität von GBSSI-Proteinen und zur Verringerung der Aktivität von BEI-Proteinen führen;
b) aus der gemäß Schritt a) hergestellten Zelle eine Kartoffelpflanze regeneriert wird; und gegebenenfalls aus der gemäß Schritt b) erzeugten Kartoffelpflanze weitere Kartoffelpflanzen erzeugt werden.

7. Vermehrungsmaterial von Kartoffelpflanzen nach Anspruch 5, enthaltend Kartoffel-Pflanzenzellen nach einem oder mehreren der Ansprüche 1 bis 4.

8. Verwendung eines oder mehrerer fremder Nucleinsäuremoleküle, die Proteine mit der enzymatischen Aktivität von GBSSI- und BE-Proteinen codieren oder deren Fragmente zur Herstellung von Kartoffel-Pflanzenzellen nach einem oder mehreren der Ansprüche 1 bis 4 oder von Kartoffelpflanzen nach Anspruch 5.

9. Verfahren zur Herstellung einer modifizierten Kartoffel-Stärke umfassend die Extraktion der Stärke aus einer Kartoffel-Pflanzenzelle nach einem oder mehreren der Ansprüche 1 bis 4 oder aus einer Kartoffelpflanze nach Anspruch 5 oder aus Vermehrungsmaterial nach Anspruch 7.

10. Verfahren nach Anspruch 9, **dadurch gekennzeichnet, dass** die Kartoffel-Stärke einen Amylopektingehalt von mindestens 90% und im Vergleich zu Stärke aus Kartoffel-Pflanzenzellen von entsprechenden Kartoffelpflanzen des waxy-Phänotyps eine um mindestens 0.5°C verringerte Verkleisterungstemperatur aufweist.

11. Verfahren nach Anspruch 9, **dadurch gekennzeichnet, dass** die Kartoffel-Stärke einen Amylopektingehalt von mindestens 90% und im Vergleich zu Stärke aus Kartoffel-Pflanzenzellen von entsprechenden Kartoffelpflanzen des waxy-Phänotyps eine um mindestens 1.5°C verringerte Verkleisterungstemperatur aufweist.

12. Verfahren nach einem der Ansprüche 9-11, **dadurch gekennzeichnet, dass** die Kartoffel-Stärke einen um mindestens 30% erhöhten Phosphatgehalt aufweist im Vergleich zu Kartoffel-Stärke aus entsprechenden Kartoffel-Wildtyppflanzen.

**Claims**

1. Transgenic potato plant cell which is genetically modified, the genetic modification consisting in the presence of one or more foreign nucleic acid molecules, whose expression leads to a decrease in the activity of GBSSI and BE proteins, in comparison to corresponding non genetically modified potato plant cells of wild-type plants, the transgenic potato plant cell containing a modified starch having an amylopectin content of at least 90% and in comparison to starch from potato plant cells of corresponding potato plants of the waxy phenotype having a gelatinization temperature decreased by at least 0.5°C and in which said foreign nucleic acid molecules are recombinant nucleic acid molecules which consist of various elements whose combination or specific spatial arrangement of which does not occur naturally in potato cells, and in which said nucleic acid molecules are selected from the group consisting of

a) DNA molecules which encode at least one antisense RNA which brings about a decrease in the expression of endogenous genes encoding GBSSI and/or BE proteins, the DNA molecules comprising either the entire sequence coding for a GBSSI protein and/or a BE protein, or parts thereof;
b) DNA molecules which lead, via a cosuppression effect, to a decrease in the expression of endogenous genes encoding GBSSI and/or BE proteins, the DNA molecules comprising either the entire sequence coding for a GBSSI protein and/or a BE protein, or parts thereof;

c) DNA molecules which encode at least one ribozyme which specifically cleaves transcripts of endogenous genes encoding GBSSI and/or BE proteins; and

d) nucleic acid molecules, introduced by means of in-vivo mutagenesis, which lead to a mutation or insertion of a heterologous sequence in endogenous genes encoding GBSSI and/or BE protein, the mutation or insertion bringing about a decrease in the expression of GBSSI and/or BE genes or the synthesis of inactive GBSSI and/or BE proteins.

2. Transgenic potato plant cell according to claim 1, in which the presence and/or the expression of one or more foreign nucleic acid molecules leads to the inhibition of the expression of endogenous genes which encode GBSSI and BE proteins.

3. Transgenic potato plant cell according to one or more of claims 1 to 2, said BE protein being a BEI protein and/or the BE gene being a BEI gene.

4. Transgenic potato plant cell according to one or more of claims 1 to 3, wherein the gelatinization temperature is decreased by at least 1.5°C in comparison to starch from potato plant cells of corresponding potato plants of the waxy phenotype.

5. Transgenic potato plant containing potato plant cells according to one or more of claims 1 to 4.

6. Process for the production of a transgenic potato plant whose starch has an amylopectin content of at least 90% and a gelatinization temperature decreased by at least 0.5°C in comparison to starch from potato plants of corresponding potato plants of the waxy phentotype, wherein

a) a potato plant cell is genetically modified by the introduction of one or more foreign nucleic acid molecules as defined in claim 1 whose presence and/or expression lead/leads to a decrease in the activity of GBSSI proteins and to a decrease in the activity of BEI proteins;

b) a potato plant is regenerated from the cell produced according to step a); and, if appropriate, further potato plants are produced from the potato plant produced according to step b).

7. Reproductive material of potato plants according to claim 5, containing potato plant cells according to one or more of claims 1 to 4.

8. Use of one or more foreign nucleic acid molecules which encode proteins having the enzymatic activity of GBSSI and BE proteins or their fragments for the production of potato plant cells according to one or more of claims 1 to 4 or of potato plants according to claim 5.

9. Process for the production of a modified potato starch comprising the extraction of the starch from a potato plant cell according to one or more of claims 1 to 4 or from a potato plant according to claim 5 or from reproductive material according to claim 7.

10. Process according to claim 9, wherein the potato starch has an amylopectin content of at least 90% and a gelatinization temperature decreased by at least 0.5°C in comparison to starch from potato plant cells of corresponding potato plants of the waxy phenotype.

11. Process according to claim 9, wherein the potato starch has an amylopectin content of at least 90% and a gelatinization temperature decreased by at least 1.5°C in comparison to starch from potato plant cells of corresponding potato plants of the waxy phenotype.

12. Process according to any one of claims 9-11, wherein the potato starch has a phosphate content increased by at least 30% in comparison to potato starch from corresponding potato wild-type plants.

**Revendications**

1. Cellule végétale transgénique de pomme de terre, qui est modifiée génétiquement, la modification génétique étant la présence d'une ou de plusieurs molécules d'acide nucléique étrangères, dont l'expression conduit à la diminution de l'activité de protéines GBSSI et BE par rapport aux cellules végétales de pomme de terre non génétiquement modifiées correspondantes de plantes de type sauvage, la cellule végétale transgénique de pomme de terre contenant un amidon modifié avec une teneur en amylopectine d'au moins 90% et présentant une température de gélatinisation diminuée d'au moins 0,5°C par rapport à l'amidon de cellules végétales de pomme de terre de plantes de pomme de terre correspondantes du phénotype cireux et dans laquelle lesdites molécules d'acide nucléique étrangères sont des molécules d'acide nucléique recombinantes, qui sont constituées de différents éléments dont la combinaison ou la disposition spatiale spécifique n'existe pas de manière naturelle dans les cellules de pomme de terre et dans laquelle lesdites molécules d'acide nucléique sont choisies dans le groupe constitué par

a) les molécules d'ADN qui codent pour au moins un ARN antisens, qui provoque une diminution de l'expression de gènes endogènes codant pour des protéines GBSSI et/ou BE, les molécules d'ADN comprenant soit la totalité de la séquence codant pour une protéine GBSSI et/ou BE, soit des parties de celle-ci ;

b) les molécules d'ADN qui conduisent, via un effet de co-suppression, à une diminution de l'expression de gènes endogènes codant pour des protéines GBSSI et/ou BE, les molécules d'ADN comprenant soit la totalité de la séquence codant pour une protéine GBSSI et/ou BE, soit des parties de celle-ci ;

c) les molécules d'ADN qui codent pour au moins un ribozyme qui clive des transcrits spécifiques de gènes endogènes, qui codent pour les protéines GBSSI et/ou BE ; et

d) les molécules d'acide nucléique introduites au moyen d'une mutagenèse in vivo, qui conduisent à une mutation ou une insertion d'une séquence hétérologue dans des gènes endogènes codant pour une protéine GBSSI et/ou BE, la mutation ou l'insertion provoquant une diminution de l'expression des gènes GBSSI et/ou BE ou la synthèse de protéines GBSSI et/ou BE inactives.

2. Cellule végétale transgénique de pomme de terre selon la revendication 1, dans laquelle la présence et/ou l'expression d'une ou de plusieurs molécules d'acide nucléique endogènes conduit à l'inhibition de l'expression de gènes endogènes, qui codent pour des protéines GBSSI et BE.

3. Cellule végétale transgénique de pomme de terre selon l'une ou plusieurs des revendications 1 à 2, où ladite protéine BE est une protéine BEI et/ou le gène BE est un gène BEI.

4. Cellule végétale transgénique de pomme de terre selon l'une ou plusieurs des revendications 1 à 3, dans laquelle la température de gélatinisation est diminuée d'au moins 1,5°C par rapport à l'amidon de cellules végétales de pommes de terre de plantes de pomme de terre correspondantes du phénotype cireux.

5. Plante transgénique de pomme de terre contenant des cellules végétales de pomme de terre selon l'une ou plusieurs des revendications 1 à 4.

6. Procédé pour la préparation d'une plante de pomme de terre transgénique, dont l'amidon présente une teneur en amylopectine d'au moins 90% et qui présente une température de gélatinisation diminuée d'au moins 0,5°C par rapport à l'amidon de plantes de pomme de terre provenant de plantes de pomme de terre correspondantes du phénotype cireux, dans lequel

a) une cellule végétale de pomme de terre est modifiée génétiquement par l'introduction d'une ou de plusieurs molécules d'acide nucléique étrangères telles que définies dans la revendication 1, dont la présence et/ou l'expression conduit à une diminution de l'activité de protéines GBSSI et à une diminution de l'activité de protéines BEI ;

b) une plante de pomme de terre est régénérée à partir de la cellule produite selon l'étape a) : et le cas échéant d'autres plantes de pomme de terre sont obtenues à partir de la plante de pomme de terre obtenue selon l'étape b).

7. Matériau de multiplication de plantes de pomme de terre selon la revendication 5, contenant des cellules végétales de pomme de terre selon l'une ou plusieurs des revendications 1 à 4.

8. Utilisation d'une ou de plusieurs molécules d'acide nucléique étrangères qui codent pour des protéines avec une activité enzymatique de protéines GBSSI et BE ou leurs fragments pour la préparation de cellules végétales de pomme de terre selon l'une ou plusieurs des revendications 1 à 4 ou de plantes de pomme de terre selon la revendication 5.

9. Procédé pour la préparation d'un amidon modifié de pomme de terre comprenant l'extraction de l'amidon d'une cellule végétale de pomme de terre selon l'une ou plusieurs des revendications 1 à 4 ou d'une plante de pomme de terre selon la revendication 5 ou de matériau de multiplication selon la revendication 7.

10. Procédé selon la revendication 9, **caractérisé en ce que** l'amidon de pomme de terre présente une teneur en amylopectine d'au moins 90% et une température de gélatinisation diminuée d'au moins 0,5°C par rapport à l'amidon de cellules végétales de pomme de terre de plantes de pomme de terre correspondantes du phénotype cireux.

11. Procédé selon la revendication 9, **caractérisé en ce que** l'amidon de pomme de terre présente une teneur en amylopectine d'au moins 90% et une température de gélatinisation diminuée d'au moins 1,5°C par rapport à l'amidon de cellules végétales de pomme de terre de plantes de pomme de terre correspondantes du phénotype cireux.

12. Procédé selon l'une quelconque des revendications 9-11, **caractérisé en ce que** l'amidon de pomme de terre présente une teneur en phosphate augmentée d'au moins 30% par rapport à l'amidon de pomme de terre de plantes de pomme de terre correspondantes de type sauvage.

## RVA-Profil

EP 1 203 087 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9711188 A1
- EP 0321201 B1
- WO 9515972 A1
- EP 120516 A
- US 5656496 A
- US 5639952 A
- US 5034322 A
- US 4962028 A
- WO 9307279 A1
- WO 9101373 A1

**In der Beschreibung aufgeführte Nicht-Patentliteratur**

- **YOUNG, A.H.** Starch Chemistry and Technology. Academic Press, 1984, 249-283
- **AKASUKA ; NELSON.** *J. Biol. Chem.,* 1966, vol. 241, 2280-2285
- **SHURE et al.** *Cell,* 1983, vol. 35, 225-233
- **HOVENKAMP-HERMELINK et al.** *Theor. Appl. Genet.,* 1987, vol. 225, 217-221
- **VISSER et al.** *Starch/Stärke,* 1997, vol. 49, 438-443
- **SAFFORD et al.** *Carbohydrate Polymers,* 1998, vol. 35, 155-168
- Starch Chemistry and Technology. Academic Press, 1988, 349-364
- **VISSER et al.** *Mol. Gen. Genet.,* 1991, vol. 225, 289-296
- **SMITH-WHITE ; PREISS.** *Plant Mol Biol. Rep.,* 1994, vol. 12, 67-71
- **LARSSON et al.** *Plant Mol Biol.,* 1998, vol. 37, 505-511
- **BABA et al.** *Biochem. Biophys. Res. Commun,* 1991, vol. 181 (1), 87-94
- **KIM et al.** *Gene,* 1998, vol. 216, 233-243
- **LARSSON et al.** *Plant Mol. Biol.,* 1998, vol. 37, 505-511
- **KHOSHNOODI et al.** *Eur. J. Biochem.,* 1996, vol. 242 (1), 148-155
- **KOSSMANN et al.** *Mol. Gen. Genet.,* 1991, vol. 230, 39-44
- **JORGENSEN.** *Trends Biotechnol.,* 1990, vol. 8, 340-344
- **NIEBEL et al.** *Curr. Top. Microbiol. Immunol.,* 1995, vol. 197, 91-103
- **FLAVELL et al.** *Curr. Top. Microbiol. Immunol.,* 1995, vol. 197, 43-46
- **PALAQUI ; VAUCHERET.** *Plant. Mol. Biol.,* 1995, vol. 29, 149-159
- **VAUCHERET et al.** *Mol. Gen. Genet.,* 1995, vol. 248, 311-317
- **DE BORNE et al.** *Mol. Gen. Genet.,* 1994, vol. 243, 613-621
- **KARTOFFEL ; KOSSMANN et al.** *Mol. Gen. Genet.,* 1991, vol. 230, 39-44
- **FEYTER et al.** *Mol. Gen. Genet.,* 1996, vol. 250, 329-338
- **KIPP, P.B. et al.** *5th International Congress of Plant Molecular Biology,* 21. September 1997
- **R. A. DIXON ; C.J. AMTZEN.** Metabolic Engineering in Transgenic Plants. Copper Mountain, CO, 1997, vol. 15, 441-447
- **KRE et al.** *Hepatology,* 1997, vol. 25, 1462-1468
- **COLE-STRAUSS et al.** *Science,* 1996, vol. 273, 1386-1389
- **OWEN.** *Bio/Technology,* 1992, vol. 10, 790-4
- **FRANKEN, E ; TEUSCHEL, U. ; HAIN, R.** *Current Opinion in Biotechnology,* 1997, vol. 8, 411-416
- **WHITELAM.** *Trends Plant Sci.,* 1996, vol. 1, 268-272
- **VON KUIPERS et al.** *Plant Mol. Biol.,* 1994, vol. 26, 1759-1773
- **VON SAFFORD et al.** *Carbohydrate Polymers,* 1998, vol. 35, 155-168
- **VON HOVENKAMP-HERMELINK et al.** *Potato Research,* 1988, vol. 31, 241-246
- **SAFFORD et al.** *Carbohydrate Polymeres,* 1998, vol. 35, 155-168
- **HOEKEMA.** The Binary Plant Vector System Offsetdrukkerij Kanters B.V. 1985
- **FRALEY et al.** *Crit. Rev. Plant Sci.,* vol. 4, 1-46
- **AN et al.** *EMBO J.,* 1985, vol. 4, 277-287
- **ROCHA-SOSA et al.** *EMBO J.,* 1989, vol. 8, 29-33
- **ROCHA-SOSA et al.** *EMBO J.,* vol. 8, 23-29
- **STOCKHAUS et al.** *Proc. Natl. Acad. Sci. USA,* 1987, vol. 84, 7943-7947
- **STOCKHAUS et al.** *EMBO J.,* 1989, vol. 8, 2445-2451

- **BANSAL et al.** *Proc. Natl. Acad. Sci.USA,* 1992, vol. 89, 3654-3658
- **LEISY et al.** *Plant Mol. Biol.,* 1990, vol. 14, 41-50
- **ZHENG et al.** *Plant J.,* 1993, vol. 4, 357-366
- **YOSHIHARA et al.** *FEBS Lett.,* 1996, vol. 383, 213-218
- **WERR et al.** *EMBO J.,* 1985, vol. 4, 1373-1380
- **PEDERSEN et al.** *Cell,* 1982, vol. 29, 1015-1026
- **QUATROCCIO et al.** *Plant Mol. Biol.,* 1990, vol. 15, 81-93
- **FIEDLER et al.** *Plant Mol. Biol.,* 1993, vol. 22, 669-679
- **BÄUMLEIN et al.** *Mol. Gen. Genet.,* 1991, vol. 225, 459-467
- **GIELEN et al.** *EMBO J.,* 1989, vol. 8, 23-29
- **WHISTLER ; BEMILLER ; PASCHALL.** Starch: Chemistry and Technology. Academic Press Inc, 1994, 412-468
- **ECKHOFF et al.** *Cereal Chem.,* 1996, vol. 73, 54-57
- **NIELSEN et al.** *Plant Physiol.,* 1994, vol. 105, 11-117
- **VON AMES.** *Methods in Enzymology,* 1966, vol. VIII, 115-118
- **VON STITT et al.** *Methods in Enzymology,* 1989, vol. 174, 518-552
- **LLOYD et al.** *Biochem. J.,* 1999, vol. 338, 515-521
- **ROCH-SOSA et al.** *EMBO J.,* 1989, vol. 8, 23-29
- **BEVAN et al.** *Nucl Acids Res,* 1983, vol. 11, 369-385
- **HERGERSBERG.** Molekulare Analyse des waxy Gens aus Solanum tuberosum und Expression von waxy antisense RNA. *Kartoffeln,* 1988
- **KOSSMANN.** Klonierung und funktionelle Analyse von Genen codierend für am Kohlenhydratstoffwechsel der Kartoffel beteiligte Proteine. *Dissertation Technische,* 1992